# EUROPÄISCHE PATENTANMELDUNG

(11) **EP 0 857 491 A1**
(43) Veröffentlichungstag der Anmeldung: **12.08.1998**
(21) Anmeldenummer: 97102153.0
(22) Anmeldetag: 11.02.1997
(51) Int. Cl.: A61M 5/00, A61M 5/20, A61M 5/19

(54) **Injektionsvorrichtung und Verpackung für die Vorrichtung und Zubehör**

(71) Anmelder: RHONE-POULENC RORER GMBH, 50792 Köln (DE)
(72) Erfinder: Hager, Jörg-Christian, 50827 Köln (DE); Gebhart, Kurt Dr., 50933 Köln (DE); Löwenich, Helmut Dr., 41363 Jüchen (DE); Pastewka, Ulrich Dr., 53115 Bonn (DE)
(74) Vertreter: Döring, Wolfgang, Dr.-Ing. Patentanwälte Hauck, Graalfs, Wehnert, Döring, Siemons

(57) **Zusammenfassung**

Es wird eine Verkaufseinheit für die parenterale Applikation von flüssigen pharmazeutischen Produkten durch den Benutzer beschrieben, wobei in der Einheit (1) mindestens eine Kanüle (2), eine Vorrichtung (3) zum Ausgeben einer vorgegebenen Flüssigkeitsmenge des pharmazeutischen Produktes in Einzeldosen sowie mindestens ein mit dem flüssigen pharmazeutischen Produkt gefülltes Gefäß (8), das in die Vorrichtung (3) einsetzbar und mittels der Vorrichtung (3) in Einzeldosen entleerbar ist, enthalten ist, und wobei jede Einzeldosis der vorgegebenen Flüssigkeitsmenge des pharmazeutischen Produktes entspricht.

## Beschreibung

Die vorliegende Erfindung betrifft eine Verkaufseinheit für die parenterale Applikation von flüssigen pharmazeutischen Produkten, eine Vorrichtung zur Durchführung dieser parenteralen Applikation und eine Nachfülleinheit für die zuvor genannte Verkaufseinheit bzw. Vorrichtung.

Parenteral applizierbare flüssige pharmazeutische Produkte, d.h. solche Produkte, die mittels Spritzen subkutan angewendet werden, werden heute überwiegend von entsprechend medizinisch geschultem Fachpersonal, so insbesondere von Ärzten oder vom Krankenpflegepersonal, vorgenommen. Hierbei wird dann eine übliche Spritze verwendet, die vor der parenteralen Applikation mit dem jeweiligen flüssigen pharmazeutischen Produkt gefüllt wird oder die bereits als Einwegspritze mit diesem flüssigen pharmazeutischen Produkt gefüllt ist. Dies bedeutet jedoch, daß sich der jeweilige Benutzer in ärztliche Behandlung begeben muß, was insbesondere dann sehr aufwendig ist, wenn täglich über einen vorgegebenen begrenzten Zeitraum hin zur Therapie der jeweiligen Erkrankung eine vorgegebene Menge des flüssigen pharmazeutischen Produktes subkutan injiziert werden muß.

Der vorliegenden Erfindung liegt die Aufgabe zugrunde, eine Möglichkeit zu schaffen, durch die der jeweilige Benutzer ein flüssiges pharmazeutisches Produkt parenteral und vorzugsweise subkutan besonders einfach applizieren kann.

Diese Aufgabe wird erfindungsgemäß durch eine Verkaufseinheit mit den Merkmalen des Patentanspruchs 1, durch eine Vorrichtung mit den Merkmalen des Patentanspruchs 11 sowie durch eine die Verkaufseinheit gemäß Patentanspruch 1 bzw. die Vorrichtung gemäß Patentanspruch 11 ggf. ergänzende Nachfülleinheit mit den Merkmalen des Patentanspruchs 33 gelöst.

Die erfindungsgemäße Verkaufseinheit für die parenterale Applikation von flüssigen pharmazeutischen Produkten durch den Benutzer während der erforderlichen Therapiezeit beruht auf dem Grundgedanken, daß dem Benutzer während der ärztlich verschriebenen Behandlungszeit alle notwendigen Teile zur Verfügung gestellt werden, so daß der Benutzer in die Lage versetzt wird, sich selbst und ohne Hilfe von medizinischem Fachpersonal das erforderliche flüssige pharmazeutische Produkt parenteral und vorzugsweise subkutan zu applizieren. Von daher umfaßt die erfindungsgemäße Verkaufseinheit mindestens eine Kanüle, eine Vorrichtung zum Ausgeben einer vorgegebenen Flüssigkeitsmenge des pharmazeutischen Produktes in Einzeldosen sowie mindestens ein mit dem flüssigen pharmazeutischen Produkt gefülltes Gefäß, das in die Vorrichtung einsetzbar und mittels der Vorrichtung in Einzeldosen entleerbar ist, wobei jede Einzeldosis der vorgegebenen Flüssigkeitsmenge des pharmazeutischen Produktes entspricht. Mit anderen Worten ist es von daher für den Benutzer lediglich erforderlich, die in der Einheit enthaltene Vorrichtung zum Ausgeben einer vorgegebenen Flüssigkeitsmenge mit der ebenfalls in der Einheit vorhandenen Kanüle zu versehen und desweiteren das mit dem flüssigen pharmazeutischen Produkt gefüllte Gefäß in die Ausgabevorrichtung einzusetzen, so daß unmittelbar hiernach der Benutzer sich selbst die vom Arzt vorgeschriebene Einzeldosis des pharmazeutischen Produktes parenteral und vorzugsweise subkutan applizieren kann.

Die erfindungsgemäße Verkaufseinheit weist eine Reihe von Vorteilen auf. So ist zunächst festzuhalten, daß bedingt dadurch, daß die erfindungsgemäße Verkaufseinheit alle für die parenterale Applikation erforderlichen Teile, die der Benutzer benötigt, in einer einzigen Verkaufseinheit enthält, so daß einerseits diese Teile auch zusammen in der entsprechenden Verkaufseinheit während der Behandlung beim Benutzer durch den Benutzer selbst aufbewahrt werden können und andererseits es für den verschreibenden Arzt besonders einfach gemacht wird, dieses Verkaufseinheit insgesamt zu verschreiben. Da die in der Verkaufseinheit enthaltene Ausgabevorrichtung zwangsläufig aufgrund ihrer Konstruktion für jede Einzeldosis die vorgegebene Flüssigkeitsmenge des pharmazeutischen Produktes bestimmt, kann es bei der erfindungsgemäßen Verkaufseinheit auch nicht zu einer Über- oder Unterdosierung des flüssigen pharmazeutischen Produktes kommen, so daß hierdurch die parenterale Applikation des flüssigen Pharmazeutischen Produktes durch den Benutzer besonders sicher gestaltet ist. Ferner hat der Benutzer alle die für die parenterale Applikation erforderlichen Teile geordnet und übersichtlich zur Hand, wodurch die Akzeptanz der erfindungsgemäßen Verkaufseinheit beim Benutzer erheblich verbessert ist. Desweiteren verhindert die erfindungsgemäße Verkaufseinheit ein Verschmutzen der darin vorhandenen Teile, insbesondere während des Gebrauchs ihres Gebrauchs beim Benutzer und erleichtert aufgrund ihrer Ausgestaltung als Einheit den Versand und die Lagerung, was sich besonders vorteilhaft im Handel bemerkbar macht.

Eine erste Ausführungsform der erfindungsgemäßen Verkaufseinheit sieht vor, daß die Einheit eine der Zahl der zu applizierenden Einzeldosen des pharmazeutischen Produktes entsprechende Anzahl von Kanülen aufweist. Somit wird hierdurch der Benutzer in die Lage versetzt, nach jeder parenteralen Applikation des flüssigen pharmazeutischen Produktes die hierfür verwendete Kanüle durch eine entsprechend steril verpackte Kanüle zu ersetzen, wodurch besonders sicher die Gefahr einer Infektion vermieden wird Vorzugsweise weisen die in der Verkaufseinheit vorgesehenen Kanülen dann einen solchen Aufbau auf, bei denen die eigentliche Nadel der Kanüle durch eine feste Kunststoffumhüllung geschützt ist, wobei diese feste Kunststoffumhüllung einerseits die Sterilität der Nadel sicherstellt und andererseits verhindert, daß sich der Benutzer an der Nadel ungewollt verletzt. Insbesondere enthält eine derartige Ausführungsform der Verkaufseinheit zehn der zuvor beschriebenen Kanülen, wobei dann das in dieser Verkaufseinheit vorhandene und mit dem flüssigen pharmazeutischen Produkt gefüllte Gefäß dementsprechend zehn Einzeldosen des parenteral zu applizierenden Produktes ermöglicht.

Um den Benutzer bei der parenteralen Applikation des flüssigen pharmazeutischen Produktes die Benutzung der erfindungsgemäßen Verkaufseinheit noch weiter zu erleichtern, sieht eine weitere vorteilhafte Ausgestaltung vor, daß in der Verkaufseinheit noch eine der Anzahl der Kanülen entsprechende Anzahl von Tupfern enthalten ist, wobei diese Tupfer mit einem Desinfektionsmittel, so zum Beispiel einem niedrigen Alkohol, Ethanol, Propanol genetzt sind. Um bei diesen Tupfern dann ein unerwünschtes Verdampfen des Desinfektionsmittels zu verhindern, sind diese, mit einem Desinfektionsmittel genetzten Tupfer dann einzeln in entsprechenden Folien, vorzugsweise Kunststoffolien und insbesondere Verbundfolien, verpackt, so daß der Benutzer vor der parenteralen Applikation des pharmazeutischen Produktes zunächst einen Tupfer dieser Verpackung entnimmt und hiermit die von ihm ausgesuchte Injektionsstelle desinfiziert.

Bei einer anderen Ausführungsform der erfindungsgemäßen Verkaufseinheit weist die Einheit zusätzlich noch einen Bereich auf, der zur Aufnahme von benutzten Kanülen und/oder von benutzten Tupfern dient. Hierbei ist dieser Bereich zur Aufnahme der benutzten Kanülen bzw. Tupfern vorzugsweise derart gestaltet, daß diese benutzten Kanülen bzw. Tupfer bei der Benutzung der Verkaufseinheit nicht aus der selben herausfallen können, so daß dieser Bereich insbesondere als separat verschließbarer Bereich ausgestaltet ist. Wird dieser Bereich als von der Verkaufseinheit lösbar angeordneter Bereich gestaltet, beispielsweise als verschließbare Box, so können die benutzten Kanülen und ggf. Tupfer separat, beispielsweise durch den Fachhandel entsorgt werden, sofern dies erwünscht oder aufgrund von gesetzlichen Vorschriften erforderlich ist.

Grundsätzlich kann in der erfindungsgemäßen Verkaufseinheit jedes Gefäß, daß die Vielzahl der Einzeldosen des flüssigen pharmazeutischen Produktes beinhalten, enthalten sein, sofern sichergestellt ist, daß dieses Gefäß so ausgestaltet ist, daß es in die Vorrichtung einsetzbar und mittels der Vorrichtung in Einzeldosen entleerbar ist. Besonders geeignet ist es jedoch, wenn das in Einzeldosen durch den Benutzer parenteral zu applizierende flüssige pharmazeutische Produkt in eine zylindrische oder zylinderartige Glascarpule (Spritzenzylinder aus Glas) gefüllt ist, wobei eine derartige Glascarpule dann insbesondere zwischen etwa 1,5 ml und etwa 8 ml, vorzugsweise zwischen etwa 2 ml und etwa 6 ml des flüssigen pharmazeutischen Produktes faßt.

Eine besonders vorteilhaft Weiterbildung der zuvor beschriebenen Glascarpule sieht vor, daß in der erfindungsgemäßen Verkaufseinheit eine solche Glascarpule vorgesehen ist, die fußseitig mit einer Durchstechmembrane und kopfseitig mit einem Stopfen verschlossen ist, wobei der Stopfen derart ausgestaltet ist, daß er axial innerhalb der Glascarpule mit abnehmendem Flüssigkeitsniveau in Richtung auf die Durchstechmembrane verschiebbar ist. Diese spezielle Ausgestaltung der Glascarpule weist den besonderen Vorteil auf, daß sich der Fußbereich des Stopfens in der unmittelbaren Nähe des Flüssigkeitsniveaus oder sogar in Kontakt damit befindet, so daß bei sinkendem Flüssigkeitsniveau in Folge der wiederholten Abgabe von Einzeldosen kein Luftpolster oberhalb des jeweiligen Flüssigkeitsstandes vorhanden ist. Dies trägt einerseits dazu bei, daß eine unerwünschte oxidative Veränderung des jeweiligen flüssigen pharmazeutischen Produktes auftritt, während andererseits durch diese spezielle Ausgestaltung des Stopfens wirksam verhindert wird, daß sich ein Vakuum aufgrund der abnehmenden Flüssigkeitsmenge in der Glascarpule ausbildet, wobei sich ein derartiges Vakuum negativ auf die Genauigkeit der in Einzeldosen abgegebenen Flüssigkeitsmenge auswirken würde.

Um bei der zuvor beschriebenen Ausführungsform der Glascarpule, die mit dem vorstehend beschriebenen axial in Richtung auf die Durchstechmembrane verschiebbaren Stopfen versehen ist, sicherzustellen, daß die erforderliche Dichtigkeit zwischen dem Stopfen und der Innenwandung der Glascarpule gewährleistet ist, sieht eine Weiterbildung der erfindungsgemäßen Verkaufseinheit vor, daß der Stopfen auf seiner Mantelfläche mindestens eine, vorzugsweise zwei bis fünf, radial nach außen weisende scheibenförmige Abschnitte aufweist. Hierbei sind diese scheibenförmige Abschnitte beim Einsetzen des Stopfens in die Glascarpule unter Ausbildung einer an der Mantelinnenfläche der Carpule flüssigkeitsdicht und luftdicht anliegenden Dichtfläche verformbar. Vorzugsweise werden die nach außen weisenden scheibenförmigen Abschnitte derart dimensioniert, daß ein Endbereich der scheibenförmigen Abschnitte durch die axiale Verschiebung des Stopfens in der Glascarpule entgegengesetzt zur Verschiebungsrichtung elastisch unter Ausbildung einer entsprechend größer dimensionierten Dichtungsfläche verformt werden. Bezüglich des Materials, aus dem der Stopfen gefertigt ist, ist festzuhalten, daß hierfür vorzugsweise ein elastisches, chemisch resistentes Kunststoff- oder Gummimaterial ausgewählt wird, wobei sich insbesondere hierfür ein Halogenbutyl-Kautschuk als besonders geeignet erwiesen hat.

Insbesondere dann, wenn der zuvor beschriebene Stopfen innerhalb der Glascarpule mit einer Kraft kleiner als 10 N, vorzugsweise mit einer Kraft zwischen 4 N und 8 N, axial verschiebbar ist, wird sichergestellt, daß die vorstehend beschriebenen Vorteile besonders reproduzierbar auftreten.

Grundsätzlich können mit Hilfe der erfindungsgemäßen Verkaufseinheit alle lagerstabilen flüssigen pharmazeutischen Produkte durch den Benutzer selbst parenteral appliziert werden. Hierbei umfaßt der Begriff flüssige pharmazeutische Produkte alle solche Produkte, die als eigentliches Produkt selbst flüssig sind oder von denen sich stabile Lösungen, stabile Dispersionen oder stabile Emulsionen in einer organischen oder anorganischen, physiologisch unbedenklichen Flüssigkeit herstellen lassen. Vorzugsweise enthält das zuvor beschriebene Gefäß oder die zuvor beschriebene bevorzugt verwendete Glascarpule eine wäßrige Lösung eines Heparins, insbesondere eine wäßrige Lösung eines niedermolekularen Heparins und speziell eine wäßrigen Lösung eines Enoxaparin-Natriums. Eine derartige Verkaufseinheit wird dann von solchen Benutzern angewendet, bei denen aufgrund des Krankheitsbildes eine längere, jedoch täglich erforderliche Heparin-Gabe in konstanten Einzeldosen aus medizinischer Sicht notwendig ist, um so beispielsweise die Thrombosengefahr auch nach Entlassung des Patienten aus dem Krankenhaus zu unterbinden. Als weitere bevorzugte Verwendung der erfindungsgemäßen Verkaufseinheit ist die regelmäßige parenterale Applikation einer vorgegebenen und konstanten Menge eines Wachstumshormones zu sehen, wobei für diesen Anwendungsfall dann das Gefäß und insbesondere die zuvor beschriebene Glascarpule mit einer Lösung, vorzugsweise einer wäßrigen Lösung, des Wachstumshormones, gefüllt ist. Hierbei wird im Rahmen der vorliegenden Anmeldung unter dem Begriff Wasser nicht nur entionisiertes oder destilliertes Wasser sondern auch alle wäßrigen Systeme, so insbesondere physiologische Salzlösungen oder Pufferlösungen, verstanden.

Die vorliegende Erfindung betrifft desweiteren eine Vorrichtung zum Ausgeben einer vorgegebenen Flüssigkeitsmenge eines pharmazeutischen Produktes in Einzeldosen, wobei die erfindungsgemäße Vorrichtung einen innerhalb eines Gehäuses angeordneten Bereich zur Aufnahme des mit dem flüssigen pharmazeutischen Produkt gefüllten Gefässes aufweist. Desweiteren ist die erfindungsgemäße Vorrichtung mit einem Abgabebereich für die Einzeldosis des pharmazeutischen Produktes versehen, wobei der Abgabebereich dann zum jeweiligen Benutzer hin weist, wenn dieser sich die Einzeldosis des pharmazeutischen Produktes selbst parenteral und insbesondere subkutan appliziert. Hierbei ist bei der erfindungsgemäßen Vorrichtung das Gefäß, in dem die Vielzahl der Einzeldosen beinhaltet ist, mit dem Abgabebereich über einen Zwischenspeicher verbindbar, derart, daß im eingesetzten Zustand des Gefässes in die erfindungsgemäße Vorrichtung die vorgegebene Flüssigkeitsmenge einer jeden Einzeldosis zunächst aus dem Gefäß in den Zwischenspeicher und dann aus dem Zwischenspeicher in den Abgabebereich überführbar ist. Über das Volumen des Zwischenspeichers ist bei der erfindungsgemäßen Vorrichtung die bei jeder Einzeldosis abgegebenen Menge des flüssigen pharmazeutischen Produktes bestimmbar. Mit anderen Worten ist somit bei der erfindungsgemäßen Vorrichtung durch Variation des Volumens des Zwischenspeichers die durch die erfindungsgemäße Vorrichtung parenteral applizierbare Menge der Einzeldosis variierbar, wobei vorzugsweise das Volumen des Zwischenspeichers derart ausgewählt wird, daß jede Einzeldosis des zu applizierenden flüssigen pharmazeutischen Produktes ein Volumen zwischen 0,1 ml und 1 ml, insbesondere zwischen 0,2 ml und 0,6 ml und vorzugsweise zwischen 0,2 ml und 0,4 ml, ausmacht.

Die erfindungsgemäße Vorrichtung weist eine Reihe von Vorteilen auf. So ist zunächst festzuhalten, daß sie einen relativ einfachen und unkomplizierten Aufbau aufweist und insbesondere sehr einfach handhabbar ist, da es zur Anwendung der erfindungsgemäßen Vorrichtung durch den Benutzer lediglich erforderlich ist, das Gefäß mit der darin enthaltenen Vielzahl von Einzeldosen des flüssigen pharmazeutischen Produktes in dem in der Vorrichtung vorgesehenen Bereich zur Aufnahme des Gefässes anzuordnen und ggf. dort zu arretieren ist, so daß dann innerhalb der Vorrichtung selbst die Verbindung des Gefässes mit dem Zwischenspeicher und von dort mit dem Ahgabebereich erfolgt. Mit anderen Worten braucht somit der jeweilige Benutzer im Unterschied zu der eingangs beschriebenen Vorgehensweise bei der parenteralen Applikation eines flüssigen pharmazeutischen Produktes durch den Arzt oder das medizinische Fachpersonal bei Anwendung der erfindungsgemäßen Vorrichtung nicht das jeweils zu applizierende Produkt aus der Ampulle in eine Spritze zu überführen und dort die genau zu injizierende Menge abzulesen, da dieser Vorgang bei der erfindungsgemäßen Vorrichtung aufgrund deren Konstruktion zwangsläufig erfolgt. Dies stellt eine enorme Erleichterung bei der Anwendung der erfindungsgemäßen Vorrichtung dar, so daß selbst kranke und/oder alte Patienten in der Lage sind, sich selbst mit Hilfe der erfindungsgemäßen Vorrichtung die erforderliche Einzeldosis des parenteral zu applizierenden Produktes zu verabreichen, ohne dabei auf fremde Hilfe angewiesen zu sein.

Um bei der erfindungsgemäßen Vorrichtung die zuvor angesprochene Verbindung zwischen dem in die Vorrichtung eingesetzten Gefäß und dem Zwischenspeicher in besonders einfacher Weise auszugestalten, sieht eine Weiterbildung der erfindungsgemäßen Vorrichtung vor, daß zwischen dem eingesetzten Gefäß, das mit der Vielzahl der Einzeldosen des flüssigen Pharmazeutischen Produktes gefüllt ist und dem Zwischenspeicher mindestens ein Verbindungskanal vorgesehen ist. Hierbei ragt vorzugsweise ein Endbereich dieses Verbindungskanals in den innerhalb des Gehäuses der erfindungsgemäßen Vorrichtung angeordneten Bereich zur Aufnahme des mit dem pharmazeutischen Produktes gefüllten Gefässes, so daß über diesen Endbereich beim Einsetzen des Gefässes die Verbindung des Zwischenspeichers mit dem eingesetzten Gefäß erleichtert wird. Wird dabei als Gefäß die zuvor bei der erfindungsgemäßen Verkaufseinheit beschriebene Glascarpule, die insbesondere fußseitig mit der zuvor bereits erwähnten Durchstechmembrane versehen ist, verwendet, so bietet es sich an, diesen Endbereich des Verbindungskanals als entsprechend angespitzter Endbereich oder vorzugsweise als Hohlnadel auszubilden, so daß durch diesen angespitzten Endbereich bzw. durch diese Hohlnadel dann die Durchstechmembrane durchstochen und somit in besonders einfacher Weise die zuvor angesprochene Verbindung zwischen dem Inhalt der Glascarpule und dem Zwischenspeicher herstellbar ist. Ein derartiges Durchstechen der Durchstechmembrane kann auch dadurch erleichtert werden, daß dem angespitzten Endbereich bzw. der Hohlnadel ein Arretierungselement für die Carpule zugeordnet ist, wobei durch dieses Arretierungselement vorzugsweise der Carpulenhals und somit auch die gesamte Carpule in ihrer Lage fixiert wird. Ist dieser Arretierungsbereich dabei als Gewinde oder als Bajonettverschluß ausgebildet, wobei das Gewinde bzw. der Bajonettverschluß mit einem entsprechend geformten Abschnitt des Carpulenhalses in Eingriff bringbar ist, so erfolgt beim Eindrehen bzw. beim Arretieren der Carpule in den Arretierungsbereich zwangsläufig eine axiale Bewegung der Carpule in Richtung auf den Verbindungskanal, so daß hierdurch der angespitzte Endbereich bzw. der als Hohlnadel ausgebildete Endbereich die Durchstechmembrane durchdringt und somit ein Strömungsweg für das flüssige pharmazeutische Produkt aus der Glascarpule in den Zwischenspeicher geschaffen wird.

Bei einer Weiterbildung der zuvor beschriebenen Ausführungsform der erfindungsgemäßen Vorrichtung ist dem Verbindungskanal ein Ventil zugeordnet, das einen Flüssigkeitsstrom vom Zwischenspeicher zurück zum Gefäß verhindert. Mit anderen Worten weist somit diese Ausführungsform der erfindungsgemäßen Vorrichtung einen Verbindungskanal auf, der mit einem Rückschlagventil versehen ist, so daß die jeweils parenteral zu applizierende Flüssigkeit den Verbindungskanal nur in eine einzige Strömungsrichtung, nämlich vom Gefäß zum Zwischenspeicher, durchströmen kann.

Bezüglich der Ausgestaltung des zuvor genannten Ventils, das, wie bereits vorstehend beschrieben ist, einen Rückfluß des flüssigen pharmazeutischen Produktes aus dem Zwischenspeicher in das Gefäß verhindert, bestehen mehrere Möglichkeiten. So kann dieses Ruckschlagventil beispielsweise als übliches Ballstauventil ausgestaltet sein. Besonders geeignet ist es jedoch, wenn das Rückschlagventil als Membranventil ausgebildet ist, wobei eine Ventilscheibe, vorzugsweise aus einem gummielastischem Material, zwischen einer ersten Stellung, in der die Ventilscheibe den zum Gefäß weisenden Teilkanalabschnitt des Verbindungskanales abdeckt, und einer zweiten Stellung, in der die Ventilscheibe in Richtung auf den Zwischenspeicher verlagert ist und somit den Flüssigkeitsstrom vom Gefäß zum Zwischenspeicher ermöglicht, bewegbar ist. Um eine Verklebung der kreisförmigen Ventilscheibe in der ersten Stellung zu verhindern und somit eine Störung der Arbeitsweise des Rückschlagventils zu vermeiden, bietet es sich an, ein ringförmiges Distanzelement vorzusehen, in das die Ventilscheibe in ihrer ersten Stellung anliegt, so daß insgesamt gesehen die Dichtfläche durch das ringförmige Distanzelement verkleinert wird, wie dies nachfolgend noch anhand eines Ausführungsbeispiels im Detail erläutert ist.

Um bei der erfindungsgemäßen Vorrichtung den Zwischenspeicher mit der Einzeldosis des flüssigen Pharmazeutischen Produktes aus dem Gefäß zu füllen, kann dieses Befüllen dadurch geschehen, daß der Inhalt des Gefässes aufgrund von Schwerkraft in den Zwischenspeicher gelangt und diesen füllt. Besonders geeignet ist es jedoch, wenn innerhalb des Zwischenspeicher ein axial in Richtung auf den Abgabebereich verschiebbarer Kolben vorgesehen ist, wobei der Kolben dann bei einer derartigen axialen Verschiebung in Richtung auf den Abgabebereich die in dem Zwischenspeicher enthaltene Einzeldosis des flüssigen pharmazeutischen Produktes zum Abgabebereich hin bewirkt. Bei einer hierzu entgegengesetzten axialen Verschiebung wird dann für den Fall, daß die Verbindung zwischen dem Zwischenspeicher und dem Abgabebereich luftdicht verschlossen ist, im Zwischenspeicher ein Vakuum erzeugt, so daß ein sich durch den Kolben erstreckender Verbindungskanal, der zwischen dem Gefäß und dem Zwischenspeicher vorgesehen ist, den Zwischenspeicher mit Flüssigkeit aus dem mit flüssigen pharmazeutischen Produkt gefüllten Gefäß füllt. Eine derartige Befüllung des Zwischenspeichers hat den besonderen Vorteil, daß hierdurch reproduzierbar eine exakt vorgegebenen Flüssigkeitsmenge aus dem Gefäß in den Zwischenspeicher gelangt, so daß dementsprechend die vom Benutzer aus dem Zwischenspeicher zu applizierende Einzeldosis des flüssigen pharmazeutischen Produktes besonders genau und reproduzierbar gestaltet werden kann.

Um bei der erfindungsgemäßen Vorrichtung die zuvor für das Befüllen erforderliche luftdichte Absperrung des zwischen dem Zwischenspeicher und dem Abgabebereich vorgesehene Kanals zu erreichen, bestehen mehrere Möglichkeiten. So kann beispielsweise in diesem Kanal ebenfalls ein Ventil, insbesondere ein Rückschlagventil, vorgesehen sein, das lediglich einen Flüssigkeitsstrom vom Zwischenspeicher zum Abgabebereich, jedoch keinen Luft- oder Flüssigkeitsstrom vom Abgabebereich zum Zwischenspeicher erlaubt. Besonders geeignet ist es jedoch, wenn dem Kanal ein von außen betätigbares Verschlußelement, so zum Beispiel ein entsprechendes Ventil, zugeordnet ist.

Eine dauerhafte und besonders einfach vom jeweiligen Benutzer zu betätigende Ausführungsform der erfindungsgemäßen Vorrichtung sieht vor, daß durch das zuvor bereits benannte Verschlußelement, durch das der Kanal, der den Abgabebereich mit dem Zwischenspeicher verbindet, luftdicht verschließbar ist, als ein dem Kanal zugeordnetes lösbares Verschlußelement ausgebildet ist. Hierbei deckt ein Dichtungsbereich des Verschlußelementes die am Abgabebereich angeordnete äußere Kanalmündung luftdicht ab. Vorzugsweise ist dieses Verschlußelement als ein auf die Vorrichtung im Abgabebereich aufschraubbares oder über ein Bajonettverschluß arretierbares Verschlußelement ausgestaltet, derart, daß es lösbar mit dem Abgabebereich und/oder dem Gehäuse der Vorrichtung verbindbar ist. Soll nunmehr bei dieser Ausführungsform der erfindungsgemäßen Vorrichtung der Zwischenspeicher in der zuvor beschriebenen Weise mit der Einzeldosis des flüssigen pharmazeutischen Produktes aus dem Gefäß gefüllt werden, was bedeutet, daß ein Luftstrom von der im Abgabebereich zugeordneten Kanalmündung zum Zwischenspeicher verhindert und somit das für die Befüllung des Zwischenspeichers erforderliche Vakuum im Zwischenspeicher erzeugt wird, so wird dieses Verschlußelement auf den Abgabebereich bzw. auf die entsprechende Zone des Gehäuses aufgeschraubt, so daß der am Verschlußelement vorgesehene Dichtungsbereich mit der Kanalmündung in Eingriff tritt. Nach Befüllung des Zwischenspeicher wird dann das Verschlußelement vom Abgabebereich bzw. der Zone des Gehäuses der Vorrichtung gelöst, so daß die im Zwischenspeicher vorhandene Einzeldosis des flüssigen pharmazeutischen Produktes durch die axiale Verschiebung des Kolbens in Richtung auf den Abgabebereich und den dann offenen Kanal aus der erfindungsgemäßen Vorrichtung abgegeben werden kann.

Eine besonders hohe Reproduzierbarkeit bezüglich der in jeder Einzeldosis abgegebenen Menge des flüssigen pharmazeutischen Produktes wird bei einer solchen Ausführungsform der erfindungsgemäßen Vorrichtung dann erreicht, wenn sichergestellt ist, daß durch eine axiale Verschiebung des in dem Zwischenspeicher angeordneten Kolbens in Richtung auf den Abgabebereich der Zwischenspeicher vollständig entleerbar ist.

Durch eine derartige vollständige Entleerung wird desweiteren verhindert, daß eine unerwünschte Kristallisation oder die Abscheidung von Resten des pharmazeutischen Produktes an den Zwischenspeicher- und/oder Kolbenwandungen auftritt, wodurch einerseits die Genauigkeit der Dosiermenge bei jeder Einzeldosis und andererseits die Funktion der erfindungsgemäßen Vorrichtung gefährdet werden würde.

Eine besonders bevorzugte Ausführungsform der erfindungsgemäßen Vorrichtung, die eine störunanfällige sowie reproduzierbare Abgabe von Einzeldosen aus dem Zwischenspeicher ermöglicht, sieht vor, daß der Zwischenspeicher zylindrisch ausgebildet ist und einen sich hieran anschließenden und zum Abgabebereich weisenden konisch ausgestalteten Endbereich aufweist, wobei der Endbereich mit dem Abgabebereich über den bereits zuvor beschriebenen Kanal verbunden ist. Desweiteren ist bei dieser Ausgestaltung der Kolben in seiner Form an die Form des zuvor beschriebenen Zwischenspeichers angepaßt, so daß dementsprechend der Kolben einen zylindrischen Kolbenbereich und einen damit verbundenen, konisch gestalteten Endbereich besitzt. Erstreckt sich bei dieser Ausführungsform der erfindungsgemäßen Vorrichtung dann noch zusätzlich der Verbindungskanal, der zwischen dem Gefäß und dem Zwischenspeicher vorgesehen ist, durch den Kolbenbereich hindurch bis in die konische Kolbenspitze, ist bei einer derartigen Ausgestaltung der erfindungsgemäßen Vorrichtung nicht nur die vollständige Entleerung sondern auch die reproduzierbare Befüllung des Zwischenspeichers im hohen Maße gewährleistet.

Bezüglich der Ausgestaltung des konischen Kolbenendbereichs bestehen mehrere Möglichkeiten. So kann dieser konische Kolbenendbereich beispielsweise aus dem selben Material ausgebildet sein wie der übrige Kolben, wobei jedoch bei einer besonders vorteilhaften Ausführungsform der konische Kolbenendbereich durch ein konisch verlaufendes Mantelrohr ausgebildet wird, das an seinem dem Kolbenbereich entgegengesetzten Ende verschlossen ist. In dieses konisch ausgestaltete Mantelrohr erstreckt sich dann nahezu über dessen gesamte axiale Länge der Verbindungskanal, der zwischen dem Gefäß und dem Zwischenspeicher vorgesehen ist, wobei das konisch ausgestaltete Mantelrohr auf seiner Mantelfläche Austrittsöffnungen für die aus dem Gefäß herangeführte Flüssigkeit besitzt. Dadurch wird sichergestellt, daß der Zwischenspeicher einwandfrei mit der aus dem Gefäß stammenden Einzeldosis des flüssigen pharmazeutischen Produktes befüllt wird.

Grundsätzlich besteht bei der erfindungsgemäßen Vorrichtung die Möglichkeit, daß die im Zwischenspeicher angeordnete Einzeldosis des flüssigen pharmazeutischen Produktes durch einen hierauf ausgeübten hohen Druck direkt über den mit dem Zwischenspeicher über den Kanal verbundenen Abgabebereich parenteral appliziert wird. Besonders geeignet ist es jedoch, wenn der Abgabebereich einen Anbringungsabschnitt zur lösbaren Befestigung einer Kanüle aufweist, derart, daß der Kanal mit der Kanüle fluiddicht verbindbar ist. Somit haltert bei dieser Ausführungsform der erfindungsgemäßen Vorrichtung der Anbringungsabschnitt eine hieran lösbar zu befestigende Kanüle, die dann bei Applikation des flüssigen pharmazeutischen Produktes in den vom Benutzer ausgewählten Körperbereich eindringt und dort die parenterale Applikation des Produktes bewirkt.

Eine Weiterbildung dieser Ausführungsform der erfindungsgemäßen Vorrichtung sieht vor, daß an dem Anbringungsabschnitt zusätzlich noch das zuvor beschriebene Verschlußelement, das bei einer Befüllung des Zwischenspeichers ein Verschließen des zwischen dem Zwischenspeicher und dem Abgabebereich vorgesehenen Kanals bewirkt, lösbar anbringbar ist. Abhängig von der Ausgestaltung des Anbringungsabschnittes und des Verschlußelementes können dann entweder gleichzeitig am Anbringungsabschnitt das Verschlußelement und die Kanüle oder wahlweise am Anbringungsabschnitt das Verschlußelement oder die Kanüle befestigt werden, wobei die zuletzt genannte Möglichkeit bevorzugt ist, da hierbei eine Verletzungsgefahr beim Entfernen des Verschlußelementes durch die dann noch am Anbringungsabschnitt arretierte Kanüle vermieden wird.

Eine andere Ausführungsform der erfindungsgemäßen Vorrichtung sieht vor, daß desweiteren dem Anbringungsabschnitt ein Schutzelement zugeordnet ist, wobei dieses Schutzelement dann zumindestens teilweise oder vollständig, eine an dem Anbringungsabschnitt befestigte Kanüle abdeckt. Gleichzeitig kann dieses Schutzelement, das den jeweiligen Benutzer vor einer ungewollten Verletzung durch die Kanüle schützt, das Verschlußelement haltern oder die Halterung des Verschlußelementes am Anbringungsabschnitt zusätzlich unterstützen, so daß hierdurch dann ein Griffbereich gebildet wird, der vom Benutzer angefaßt wird, wenn dieser die erfindungsgemäße Vorrichtung zur Anwendung vorbereitet.

Eine weitere, besonders geeignete Ausführungsform der erfindungsgemäßen Vorrichtung umfaßt einen innerhalb des Gehäuses vorgesehenen ersten Zylinder und einen den ersten Zylinder umschließenden zweiten Zylinder, wobei der erste und der zweite Zylinder axial relativ zum Gehäuse verschiebbar sind. Innerhalb des ersten Zylinders ist der Bereich zur Aufnahme des Gefässes, insbesondere der Bereich zur Aufnahme der zuvor beschriebenen Carpule, ausgebildet, während innerhalb des zweiten Zylinders der mit dem Gefäß verbundene Zwischenspeicher vorgesehen ist. Hierbei sind bei dieser Ausführungsform der erfindungsgemäßen Vorrichtung der erste und der zweite Zylinder zwischen einer ersten Stellung, in der die am Anbringungsabschnitt arretierbare Kanüle über das zuvor beschriebene Schutzelement bzw. das Gehäuse der Vorrichtung hinaus vorsteht und in der der Zwischenspeicher über den Kanal und die hieran angeschlossene Kanüle entleert wurde, und einer zweiten Stellung, in der eine am Anbringungsabschnitt arretierbare Kanüle vom Schutzelement abgedeckt ist und in der der Zwischenspeicher mit der Einzeldosis gefüllt ist, sowie umgekehrt hierzu axial verschiebbar.

Diese zuvor beschriebene Ausführungsform der erfindungsgemäßen Vorrichtung erlaubt in besonders einfacher Weise eine parenterale Applikation der entsprechenden Einzeldosis des flüssigen pharmazeutischen Produktes. Hierzu ist es lediglich erforderlich, daß, ausgehend von der ersten Stellung, in der der Zwischenspeicher leer ist, der erste und der zweite Zylinder durch eine axiale Verschiebung dieser Zylinder relativ zum Gehäuse in eine zweite Stellung überführt werden. Diese axiale Verschiebung bewirkt, daß der Zwischenspeicher mit der aus dem Gefäß stammenden Flüssigkeit befüllt wird. Nach Anbringung einer entsprechenden Kanüle am Anbringungsabschnitt erfolgt dann durch axiale Verschiebung der beiden Zylinder in Richtung auf den Anbringungsabschnitt das Einstechen der Kanüle in den Körperbereich des Benutzers und das Entleeren des Zwischenspeichers und somit die parenterale Applikation der Einzeldosis. Vorzugsweise werden die beiden Zylinder jedoch so ausgestaltet, daß sie zusätzlich noch relativ zueinander axial verschiebbar sind, so daß zwischen dem eigentlichen Einstechvorgang der Kanüle in den Körperbereich des Benutzers und das Entleeren des Zwischenspeichers eine Zeitdifferenz besteht und damit ein zeitversetztes Applizieren des flüssigen pharmazeutischen Produktes ermöglicht wird.

Um die zuvor beschriebene axiale Verschiebung der beiden Zylinder aus der ersten Stellung in die zweite Stellung und aus der zweiten Stellung in die erste Stellung herbeizuführen, können diese axialen Verschiebungen in beide Richtungen manuell durchgeführt werden. Dies bedeutet jedoch, daß bei einer derartigen, vom jeweiligen Anwender herbeigeführten Überführung der beiden Zylinder von der zweiten in die erste Stellung das Eindringen der Kanüle in den Körperbereich manuell selbst herbeigeführt werden muß, was von einigen Benutzern als äußerst unangenehm und teilweise sogar als unüberwindbares Hindernis angesehen wird. Von daher sieht eine besonders vorteilhafte Weiterbildung der zuvor beschriebenen Ausführungsform der erfindungsgemäßen Vorrichtung vor, daß dem ersten und dem zweiten Zylinder jeweils eine Feder zugeordnet ist, derart, daß in der zweiten Stellung der Zylinder diese beiden Federn gespannt und in der ersten Stellung der Zylinder diese beiden Federn entspannt sind.

Um die beiden Zylinder in ihrer zweiten, durch die entsprechenden Federn gespannte Stellung zu fixieren, kann bei der erfindungsgemäßen Vorrichtung jeweils ein Rastelement für jeden Zylinder vorgesehen sein. Durch Lösen des dem zweiten Zylinder zugeordneten Rastelementes wird dann der zweite Zylinder aufgrund der Federkraft der dem zweiten Zylinder zugeordneten Feder aus der zweiten Stellung in die erste Stellung überführt, was bewirkt, daß eine am Anbringungsabschnitt arretierte Kanüle zwangsläufig und ohne Beeinflussung durch den Benutzer in den ausgewählten Körperbereich eindringt. Hiernach wird dann die dem ersten Zylinder zugeordnete Raste gelöst, wodurch der erste Zylinder aufgrund der dem ersten Zylinder zugeordneten Feder axial in Richtung auf den Abgabebereich hin bewegt und somit der Zwischenspeicher entleert wird, so daß die entsprechende Einzeldosis des flüssigen pharmazeutischen Produktes parenteral verabreicht wird.

Bei einer besonders geeigneten und vom jeweiligen Benutzer einfach und problemlos anzuwendenden Weiterbildung der zuvor beschriebenen Ausführungsform der erfindungsgemäßen Vorrichtung sind beide Zylinder über ein einziges Rastelement in der zweiten Stellung arretierbar. Dies bedeutet, daß der Benutzer nur ein einziges Rastelement lösen muß, um die in der zweiten Stellung arretierten und federbelasteten beiden Kolben gemeinsam in die erste Stellung zu überführen, wodurch dann der Einstichvorgang der Kanüle in den Körperbereich und das Injizieren der Einzeldosis erfolgt.

Um bei der zuvor beschriebenen Ausführungsform der erfindungsgemäßen Vorrichtung zu erreichen, daß zuerst der Einstich der Kanüle in den Körperbereich und hiernach dann das Injizieren der Einzeldosis erfolgt, weist eine besonders geeignete Weiterbildung dieser Ausführungsform der erfindungsgemäßen Vorrichtung Federn auf, die in ihren Federkennlinien aufeinander angepaßt sind. Hierbei besitzt die dem zweiten Zylinder zugeordnete Feder eine steilere Federkennlinie als die Feder, die am ersten Zylinder vorgesehen ist, so daß die bereits zuvor angesprochene zeitliche Verzögerung zwischen dem Einstechen der Kanüle und der anschließenden Injektion über die Abstimmung der Federkennlinien der beiden Federn in einem weiten Bereich einstellbar ist.

Bei einer weiteren, vorteilhaften Ausführungsform der erfindungsgemäßen Vorrichtung ist innerhalb des Gehäuses ein erster Zylinder und ein den ersten Zylinder umschließender zweiter Zylinder vorgesehen, wobei der erste und der zweite Zylinder axial relativ zum Gehäuse verschiebbar sind. Innerhalb des ersten Zylinders ist der Bereich zur Aufnahme des Gefässes, insbesondere der zuvor beschriebenen Glascarpule, ausgebildet und innerhalb des zweiten Zylinders ist der mit dem Gefäß verbundene Zwischenspeicher vorgesehen, derart, daß der erste und zweite Zylinder gemeinsam aus einer ersten Stellung, in der die an dem Anbringungsabschnitt arretierbare Kanüle über das Schutzelement bzw. das Gehäuse vorsteht und in der der Zwischenspeicher leer ist, in eine zweite Stellung, in der die an dem Anbringungsabschnitt arretierbare Kanüle von dem Schutzelement abgedeckt ist, und umgekehrt hierzu axial relativ zum Gehäuse verschiebbar ist. Zum Füllen des Zwischenspeichers ist der erste Zylinder relativ zur zweiten Stellung des zweiten Zylinders in eine dritte Stellung axial weiter in Richtung auf das Gefäß verschiebbar.

Mit anderen Worten weist diese zuvor beschriebene besonders vorteilhafte Ausgestaltung der erfindungsgemäßen Vorrichtung im Unterschied zu den zuvor beschriebenen Ausführungsformen der Vorrichtung, bei denen die Zylinder nur eine erste und zweite Stellung einnehmen können, noch eine dritte Stellung auf.

Ausgehend von der ersten Stellung der beiden Zylinder werden zur Benutzung der erfindungsgemäßen Vorrichtung zunächst beide Zylinder in die zweite Stellung überführt, was zur Folge hat, daß eine am Anbringungsabschnitt arretierte Kanüle von einem Schutzelement abgedeckt ist. Zu diesem Zeitpunkt ist der Zwischenspeicher noch nicht mit der Einzeldosis des flüssigen pharmazeutischen Produktes befüllt. Anschließend wird der erste Zylinder relativ zum zweiten Zylinder, der in der zweiten Stellung verbleibt, axial in Richtung auf das Gefäß in eine dritte Stellung verschoben, wodurch der Zwischenspeicher über den Verbindungskanal aus dem Gefäß mit dem flüssigen pharmazeutischen Produkt vollständig gefüllt wird. Bei dieser Überführung des ersten Zylinders aus der zweiten Stellung in die dritte Stellung ist der Kanal, der die Kanüle mit dem Zwischenspeicher verbindet, über eine der zuvor beschriebenen Ventile oder über das ebenfalls schon zuvor abgehandelte Verschlußelement luftdicht abgeschlossen. Somit bildet sich im Zwischenspeicher ein entsprechendes Vakuum aus, das den Flüssigkeitsstrom vom Gefäß zum Zwischenspeicher fördert oder bewirkt.

Die somit anwendungsbereite Vorrichtung wird dann in Kontakt mit der ausgewählten Körperzone gebracht, so daß hiernach der Benutzer den zweiten Zylinder aus der zweiten Stellung in die erste Stellung überführt, wodurch der Einstichvorgang der Kanüle in die Körperzone manuell herbeigeführt wird. Hiernach überführt der Benutzer dann den ersten Zylinder aus der dritten Stellung in die erste Stellung, wodurch die entsprechende Injektion der Einzeldosis des flüssigen pharmazeutischen Produktes aus dem Zwischenspeicher in den Körper bewirkt wird.

Wie jedoch vorstehend bereits erwähnt ist, besteht bei einer derartigen Anwendung bei einigen Benutzern das Problem, daß sie selbst den Einstichvorgang der Kanüle in ihren eigenen Körper herbeiführen müssen. Um dies zu vermeiden, sieht eine Weiterbildung der zuvor beschriebenen Ausführungsform der erfindungsgemäßen Vorrichtung vor, daß jedem Zylinder (erster Zylinder, zweiter Zylinder) jeweils eine Feder zugeordnet ist, wobei eine erste Feder die Lage des ersten Zylinders in der dritten Stellung und eine zweite Feder die Lage des zweiten Zylinders in der zweiten Stellung vorspannt. Desweiteren ist der erste Zylinder in der dritten Stellung durch eine erste Raste und der zweite Zylinder in der zweiten Stellung durch eine zweite Raste fixierbar, derart, daß diese durch entsprechende Rasten herbeigeführte Fixierung vorzugsweise durch eine einzige Auslösetaste lösbar ist.

Um eine derartige Vorrichtung zu benutzen, überführt der jeweilige Benutzer den zweiten Zylinder gegen die Kraft der entsprechenden zweiten Feder in die zweite Stellung, wobei eine derartige axiale Verschiebung des zweiten Zylinders vorzugsweise durch ein außen am Gehäuse vorgesehenes Verschiebeelement herbeigeführt wird. Gleihzeitig mit Betätigung dieses Verschiebeelementes wird der erste Zylinder gegen die Kraft der ersten Feder in seine zweite Stellung verschoben, wobei die zweite Raste dann den zweiten Zylinder in der zweiten Stellung fixiert. Bei einer weiteren Betätigung des Verschiebeelementes wird der erste Zylinder aus der zweiten Stellung in die dritte Stellung gegen die Kraft der entsprechenden ersten Feder axial relativ zum zweiten Zylinder verschoben und in der dritten Stellung durch die erste Raste fixiert.

Bei Betätigung der gemeinsamen Auslösetaste erfolgt aufgrund der Federkräfte der ersten und zweiten Feder eine axiale Verschiebung der beiden Zylinder aus der dritten Stellung (erster Zylinder) bzw. zweiten Stellung (zweiter Zylinder) in die erste Stellung, wodurch zwangsläufig und ohne Beeinflussung durch den Benutzer das Eindringen der Kanüle in den Körperbereich und das Injizieren der Einzeldosis aus dem Zwischenspeicher erfolgt.

Vorzugsweise sieht eine Weiterbildung der zuvor beschriebenen Ausführungsform vor, daß durch die Betätigung der gemeinsamen Auslösetaste zunächst die zweite Raste, die den zweiten Zylinder in der zweiten Stellung fixiert, und hierzu zeitverzögert dann erst die erste Raste, die den ersten Zylinder in seiner dritten Stellung fixiert, lösbar ist.

Insbesondere werden die zuvor beschriebenen erfindungsgemäßen Vorrichtungen in der eingangs abgehandelten Verkaufseinheit verwendet, wobei jedoch die erfindungsgemäße Vorrichtung selbst ohne die Verkaufseinheit in den Handel gelangen kann.

Desweiteren betrifft die vorliegende Erfindung eine Nachfülleinheit zur Verwendung bei der eingangs beschriebenen erfindungsgemäßen Verkaufseinheit oder bei der vorstehend abgehandelten erfindungsgemäßen Vorrichtung.

Hierbei umfaßt die erfindungsgemäße Nachfülleinheit mindestens eine Kanüle sowie mindestens ein mit dem flüssigen pharmazeutischen Produkt gefülltes Gefäß, das in die vorstehend beschriebene erfindungsgemäße Vorrichtung einsetzbar und mittels dieser Vorrichtung in Einzeldosen entleerbar ist.

Die erfindungsgemäße Nachfülleinheit weist den entscheidenden Vorteil auf, daß sie sowohl auf die vorstehend beschriebene Verkaufseinheit als auch auf die erfindungsgemäße Vorrichtung bezüglich ihrer konstruktiven Merkmale ideal angepaßt ist, so daß der Benutzer der vorstehend beschriebenen erfindungsgemäßen Verkaufseinheit oder der vorstehend abgehandelten erfindungsgemäßen Vorrichtung die erfindungsgemäße Nachfülleinheit dann benutzen kann, wenn eine längere parenterale Applikation des flüssigen pharmazeutischen Produktes aus medizinischer Sicht erforderlich ist und in Folge der regelmäßigen Benutzung das mit dem flüssigen pharmazeutischen Produkt gefüllte und in der vorstehend beschriebenen erfindungsgemäßen Verkaufseinheit enthaltene Gefäß entleert ist.

Eine erste Ausgestaltung der erfindungsgemäßen Nachfülleinheit sieht vor, daß in der Nachfülleinheit ein Demontageelement zur Entfernung des entleerten Gefässes aus der erfindungsgemäßen Vorrichtung zum Ausgeben einer vorgegebenen Flüssigkeitsmenge in Einzeldosen vorgesehen ist. Abhängig von der jeweiligen Ausgestaltung des innerhalb des Gehäuses der erfindungsgemäßen Vorrichtung angeordneten Bereichs zur Aufnahme des mit dem flüssigen pharmazeutischen Produkt gefüllten Gefässes, richtet sich auch die Ausgestaltung des Demontageelementes, wobei dieses Demontageelement vorzugsweise einen Griffabschnitt zur Erfassung des Demontageelementes durch den Benutzer und einen Arretierungsabschnitt zur formschlüssigen und/oder kraftschlüssigen Arretierung des entleerten Gefässes aufweist.

Wie bereits vorstehend schon bei der erfindungsgemäßen Verkaufseinheit beschrieben ist, sieht eine erste Ausführungsform der erfindungsgemäßen Nachfülleinheit vor, daß die Einheit eine der Zahl der zu applizierenden Einzeldosen des pharmazeutischen Produktes entsprechende Anzahl von Kanülen aufweist. Somit wird hierdurch der Benutzer in die Lage versetzt, nach jeder parenteralen Applikation des flüssigen pharmazeutischen Produktes die hierfür verwendete Kanüle durch eine entsprechend steril verpackte Kanüle zu ersetzen, wodurch besonders sicher die Gefahr einer Infektion vermieden wird. Vorzugsweise weisen die in der Nachfülleinheit vorgesehenen Kanülen dann einen solchen Aufbau auf, bei denen die eigentliche Nadel der Kanüle durch eine feste Kunststoffumhüllung geschützt ist, wobei diese feste Kunststoffumhüllung einerseits die Sterilität der Nadel sicherstellt und andererseits verhindert, daß sich der Benutzer an der Nadel ungewollt verletzt. Insbesondere enthält eine derartige Ausführungsform der Nachfülleinheit zehn der zuvor beschriebenen Kanülen, wobei dann das in dieser Nachfülleinheit vorhandene und mit dem flüssigen pharmazeutischen Produkt gefüllte Gefäß dementsprechend zehn Einzeldosen des parenteral zu applizierenden Produktes ermöglicht.

Um den Benutzer bei der parenteralen Applikation des flüssigen pharmazeutischen Produktes die Benutzung der erfindungsgemäßen Nachfülleinheit noch weiter zu erleichtern, sieht eine weitere vorteilhafte Ausgestaltung vor, daß in der Nachfülleinheit noch eine der Anzahl der Kanülen entsprechende Anzahl von Tupfern enthalten ist, wobei diese Tupfer mit einem Desinfektionsmittel, so zum Beispiel einem niedrigen Alkohol (Ethanol, Propanol), genetzt sind. Um bei diesen Tupfern dann ein unerwünschtes Verdampfen des Desinfektionsmittels zu verhindern, sind diese, mit einem Desinfektionsmittel genetzten Tupfer dann einzeln in entsprechenden Folien, vorzugsweise Kunststoffolien und insbesondere Verbundfolien, verpackt, so daß der Benutzer vor der parenteralen Applikation des pharmazeutischen Produktes zunächst einen Tupfer dieser Verpackung entnimmt und hiermit die von ihm ausgesuchte Injektionsstelle desinfiziert.

Bei einer anderen Ausführungsform der erfindungsgemäßen Nachfülleinheit weist die Einheit zusätzlich noch einen Bereich auf, der zur Aufnahme von benutzten Kanülen und/oder von benutzten Tupfern dient. Hierbei ist dieser Bereich zur Aufnahme der benutzten Kanülen bzw. Tupfern vorzugsweise derart gestaltet, daß diese benutzten Kanülen bzw. Tupfer bei der Benutzung der Nachfülleinheit nicht aus der selben herausfallen können, so daß dieser Bereich insbesondere als separat verschließbarer Bereich ausgestaltet ist. Wird dieser Bereich als von der Nachfülleinheit lösbar angeordneter Bereich gestaltet, beispielsweise als verschließbare Box, so können die benutzten Kanülen und ggf. Tupfer separat, beispielsweise durch den Fachhandel entsorgt werden, sofern dies erwünscht oder aufgrund von gesetzlichen Vorschriften erforderlich ist.

Grundsätzlich kann in der erfindungsgemäßen Nachfülleinheit jedes Gefäß, daß die Vielzahl der Einzeldosen des flüssigen pharmazeutischen Produktes beinhalten, enthalten sein, sofern sichergestellt ist, daß dieses Gefäß so ausgestaltet ist, daß es in die Vorrichtung einsetzbar und mittels der Vorrichtung in Einzeldosen entleerbar ist. Besonders geeignet ist es jedoch, wenn das in Einzeldosen durch den Benutzer parenteral zu applizierende flüssige pharmazeutische Produkt in eine zylindrische oder zylinderartige Glascarpule gefüllt ist, wobei eine derartige Glascarpule dann insbesondere zwischen etwa 1,5 ml und etwa 8 ml, vorzugsweise zwischen etwa 2 ml und etwa 6 ml des flüssigen pharmazeutischen Produktes faßt.

Eine besonders vorteilhafte Weiterbildung der zuvor beschriebenen Glascarpule sieht vor, daß in der erfindungsgemäßen Nachfülleinheit eine solche Glascarpule vorgesehen ist, die fußseitig mit einer Durchstechmembrane und kopfseitig mit einem Stopfen verschlossen ist, wobei der Stopfen derart ausgestaltet ist, daß er axial innerhalb der Glascarpule mit abnehmendem Flüssigkeitsniveau in Richtung auf die Durchstechmembrane verschiebbar ist. Diese spezielle Ausgestaltung der Glascarpule weist den besonderen Vorteil auf, daß sich der Fußbereich des Stopfens in der unmittelbaren Nähe des Flüssigkeitsniveaus oder sogar in Kontakt damit befindet, so daß bei sinkendem Flüssigkeitsniveau in Folge der wiederholten Abgabe von Einzeldosen kein Luftpolster oberhalb des jeweiligen Flüssigkeitsstandes vorhanden ist. Dies trägt einerseits dazu bei, daß eine unerwünschte oxidative Veränderung des jeweiligen flüssigen pharmazeutischen Produktes auftritt, während andererseits durch diese spezielle Ausgestaltung des Stopfens wirksam verhindert wird, daß sich ein Vakuum aufgrund der abnehmenden Flüssigkeitsmenge in der Glascarpule ausbildet, wobei sich ein derartiges Vakuum negativ auf die Genauigkeit der in Einzeldosen abgegebenen Flüssigkeitsmenge auswirken würde.

Um bei der zuvor beschriebenen Ausführungsform der Glascarpule, die mit dem vorstehend beschriebenen axial in Richtung auf die Durchstechmembrane verschiebbaren Stopfen versehen ist, sicherzustellen, daß die erforderliche Dichtigkeit zwischen dem Stopfen und der Innenwandung der Glascarpule gewährleistet ist, sieht eine Weiterbildung der erfindungsgemäßen Nachfülleinheit vor, daß der Stopfen auf seiner Mantelfläche mindestens eine, vorzugsweise zwei bis fünf, radial nach außen weisende scheibenförmige Abschnitte aufweist. Hierbei sind diese scheibenförmige Abschnitte beim Einsetzen des Stopfens in die Glascarpule unter Ausbildung einer an der Mantelinnenfläche der Carpule flüssigkeitsdicht und luftdicht anliegenden Dichtfläche verformbar. Vorzugsweise werden die nach außen weisenden scheibenförmigen Abschnitte derart dimensioniert, daß ein Endbereich der scheibenförmigen Abschnitte durch die axiale Verschiebung des Stopfens in der Glascarpule entgegengesetzt zur Verschiebungsrichtung elastisch unter Ausbildung einer entsprechend größer dimensionierten Dichtungsfläche verformt werden. Bezüglich des Materials, aus dem der Stopfen gefertigt ist, ist festzuhalten, daß hierfür vorzugsweise ein elastisches, chemisch resistentes Kunststoff- oder Gummimaterial ausgewählt wird, wobei sich insbesondere hierfür ein Halogenbutyl-Kautschuk als besonders geeignet erwiesen hat.

Insbesondere dann, wenn der zuvor beschriebene Stopfen innerhalb der Glascarpule mit einer Kraft kleiner als 10 N, vorzugsweise mit einer Kraft zwischen 4 N und 8 N, axial verschiebbar ist, wird sichergestellt, daß die vorstehend beschriebenen Vorteile besonders reproduzierbar auftreten.

Grundsätzlich können mit Hilfe der die erfindungsgemäße Nachfülleinheit in Verbindung mit der erfindungsgemäßen Vorrichtung oder der erfindungsgemäßen Verkaufseinheit alle lagerstabilen flüssigen pharmazeutischen Produkte durch den Benutzer selbst parenteral appliziert werden. Hierbei umfaßt der Begriff flüssige pharmazeutische Produkte alle solche Produkte, die als eigentliches Produkt selbst flüssig sind oder von denen sich stabile Lösungen, stabile Dispersionen oder stabile Emulsionen in einer organischen oder anorganischen, physiologisch unbedenklichen Flüssigkeit herstellen lassen. Vorzugsweise enthält das zuvor beschriebene Gefäß oder die zuvor beschriebene bevorzugt verwendete Glascarpule eine wäßrige Lösung eines Heparins, insbesondere eine wäßrige Lösung eines niedermolekularen Heparins und speziell eine wäßrigen Lösung eines Enoxaparin-Natriums.

Eine derartige Nachfülleinheit wird dann von solchen Benutzern angewendet, bei denen aufgrund des Krankheitsbildes eine längere, jedoch täglich erforderliche Heparin-Gabe in konstanten Einzeldosen aus medizinischer Sicht notwendig ist, um so beispielsweise die Thrombosengefahr auch nach Entlassung des Patienten aus dem Krankenhaus zu unterbinden. Als weitere bevorzugte Verwendung der erfindungsgemäßen Nachfülleinheit ist die regelmäßige parenterale Applikation einer vorgegebenen und konstanten Menge eines Wachstumshormones zu sehen, wobei für diesen Anwendungsfall dann das Gefäß und insbesondere die zuvor beschriebene Glascarpule mit einer Lösung, vorzugsweise einer wäßrigen Lösung, des Wachstumshormones, gefüllt ist.

Die erfindungsgemäße Verkaufseinheit, die erfindungsgemäße Vorrichtung sowie die erfindungsgemäße Nachfülleinheit werden nachfolgend anhand von Ausführungsbeispielen in Verbindung mit der Zeichnung näher erläutert. Es zeigen:
- Figur 1: eine perspektivische Ansicht einer Ausführungsform der Verkaufseinheit;
- Figur 2: eine perspektivische, schematische Darstellung der Vorrichtung zum Ausgeben einer vorgegebenen Flüssigkeitsmenge in Einzeldosen;
- Figur 3: eine Explosionszeichnung zu Figur 2;
- Figur 4: eine vergrößerte schematische Schnittansicht eines Verschlußelementes;
- Figur 5: eine Explosionsansicht im Schnitt der axial verschiebbaren Zylinder;
- Figur 6: eine Schnittansicht einer ersten Ausführungsform mit entleertem Zwischenspeicher;
- Figur 7: wie Figur 6, jedoch mit gefülltem Zwischenspeicher;
- Figur 8: eine vergrößerte schematische Schnittansicht eines Rückschlagventils;
- Figur 9: eine Schnittansicht einer zweiten Ausführungsform der Vorrichtung mit gefülltem Zwischenspeicher; und
- Figur 10: eine perspektivische Ansicht einer Ausführungsform der Nachfülleinheit.

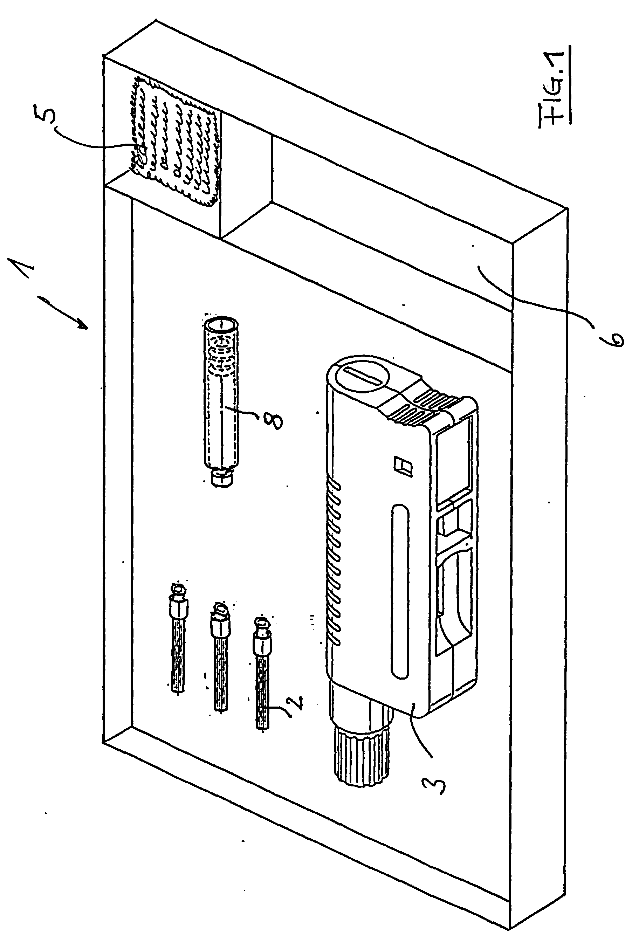

In den Figuren 1 bis 10 sind die selben Teile mit den selben Bezugsziffern beschriftet.

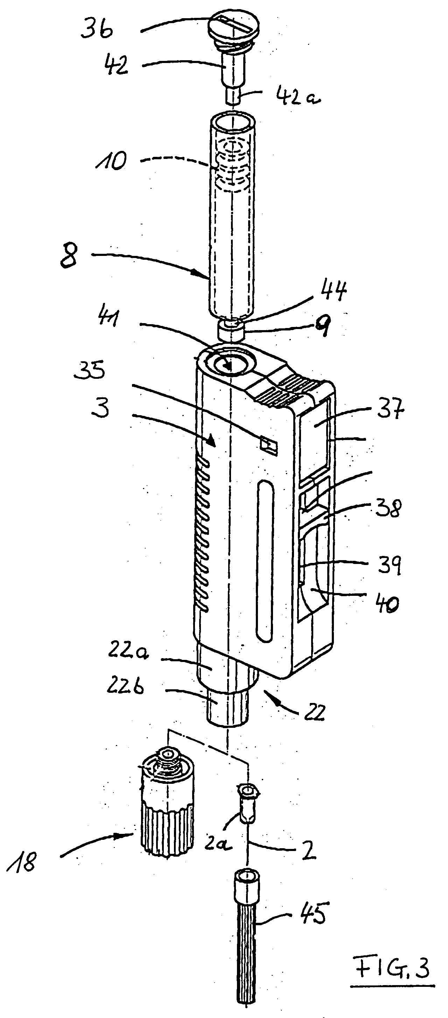

Eine insgesamt mit 1 bezeichnete und in Figur 1 dargestellte Verkaufseinheit umfaßt eine Vorrichtung 3 zum Abgeben einer vorgegebenen Flüssigkeitsmenge des pharmazeutischen Produktes in Einzeldosen, eine Vielzahl von Kanülen 2, die mit einem entsprechendem Kanülenschutz 45 (Figur 3) zur sterilen Aufbewahrung versehen sind, eine mit 8 bezeichnete Glascarpule, die als mit einem flüssigen pharmazeutischen Produkt gefülltes Gefäß dient. Desweiteren sind innerhalb der Verkaufseinheit 1 eine der Anzahl der Kanülen 2 entsprechende Anzahl von mit einem Desinfektionsmittel genetzte Tupfer 5 vorhanden. Ferner weist die Verkaufseinheit 1 einen Bereich 6 zur Aufnahme von benutzten Kanülen auf, wobei dieser Bereich bei der in der Figur 1 abgebildeten Ausführungsform mit einem nicht gezeigten Deckel verschließbar ist. Insgesamt ist dieser Bereich 6 lösbar an der Verkaufseinheit 1 angeordnet, so daß nach Aufnahme aller benutzten Kanülen dieser Bereich 6 getrennt entsorgt werden kann, sofern dies gesetzlich vorgeschrieben oder erwünscht ist. In der Verkaufseinheit 1 sind üblicherweise 10 Kanülen 2 enthalten, so daß dementsprechend die Flüssigkeitsmenge für 10 Einzeldosen in der Glascarpule 8 enthalten ist. Üblicherweise variiert die in jeder Einzeldosis abgegebene Flüssigkeitsmenge zwischen 0,1 ml und 1 ml, so daß dementsprechend in der Glascarpule zwischen 1 ml und 10 ml des flüssigen pharmazeutischen Produktes enthalten ist. Um zu vermeiden, daß das Desinfektionsmittel der Tupfer 5 verdampft, ist jeder Tupfer 5 separat und fluiddicht in einer entsprechenden Kunststoffolie verpackt. Die Verkaufseinheit 1 ist insgesamt mit einem Deckel (nicht gezeigt) verschlossen oder ist unter Aufnahme der zuvor beschriebenen Teile in der üblichen Kunststoffverpackung, ggf. sogar steril, angeordnet.

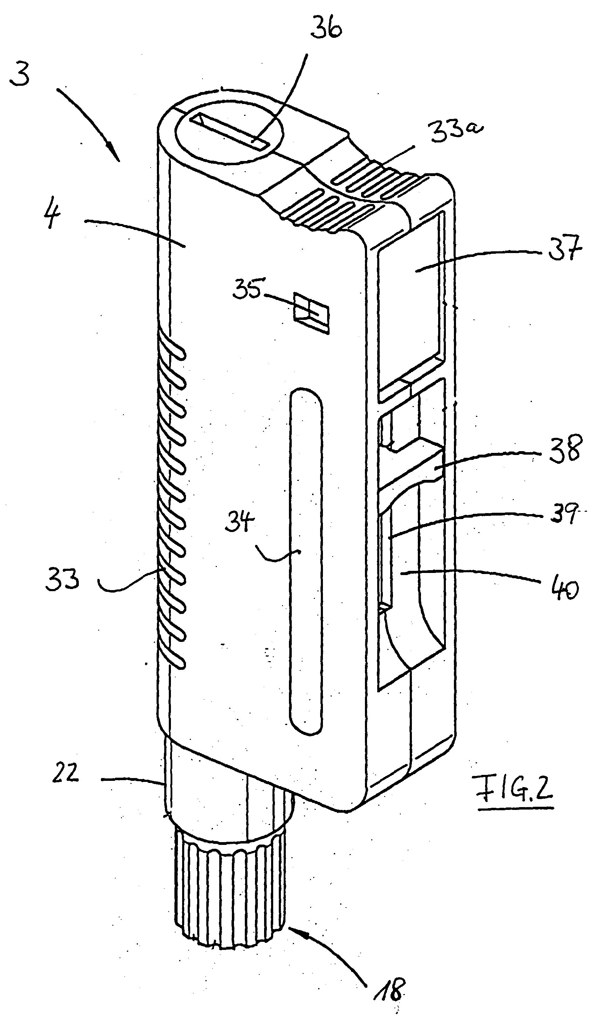

Die in Figur 2 im Detail abgebildete Vorrichtung 3 zum Ausgeben einer vorgegebenen Flüssigkeitsmenge des flüssigen pharmazeutischen Produktes weist ein Schutzelement 22 auf, wobei dieses Schutzelement 22 aus einem äußeren Zylinder 22 a und einem konzentrisch hierzu angeordneten inneren Zylinder 22 b besteht, die aus einem transparenten Kunststoffmaterial angefertigt sind und durch die eine an der Vorrichtung 3 arretierte Kanüle 2 abgedeckt werden kann, wie dies zum Beispiel in der Figur 3 erkennbar ist. Desweiteren besitzt die Vorrichtung 3 ein Gehäuse 4, wobei am Gehäuse 4 ein oberer Griffbereich 33, ein Sichtfenster 34, ein Zählwerk 35, ein zweiter Griffbereich 33 a, ein vom Gehäuse lösbarer Deckel 36, eine Auslösetaste 37, ein Verschiebeelement 38, das von einem entsprechend geformten Schlitz 39 gehaltert und geführt ist, sowie eine Vertiefung 40 vorgesehen sind.

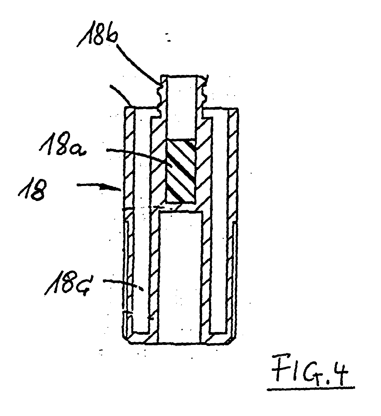

Bei der in Figur 2 gezeigten Vorrichtung 3 ist desweiteren ein Verschlußelement 18 am Gehäuse 4 arretiert, wobei das Verschlußelement 18 nachfolgend noch im Detail in Verbindung mit der Figur 4 erläutert wird. Somit ist die Vorrichtung 3 vom Benutzer an den Griffbereichen 33 und 33 a sowie an dem Verschlußelement 18 ohne Schwierigkeiten zu halten. Das zuvor angesprochene Zählwerk 35 dient zur Zählung der von der Vorrichtung 3 abgegebenen Einzeldosen des flüssigen pharmazeutischen Produktes, während das im Gehäuse vorgesehene Sichtfenster 34 eine Kontrolle des Flüssigkeitsstandes in der Glascarpule 8 ermöglicht, sofern diese von der Vorrichtung 3 aufgenommen ist, wobei zur Anordnung der Glascarpule 8 der Deckel 36 entfernt wird, wie dies in Figur 3 dargestellt ist.

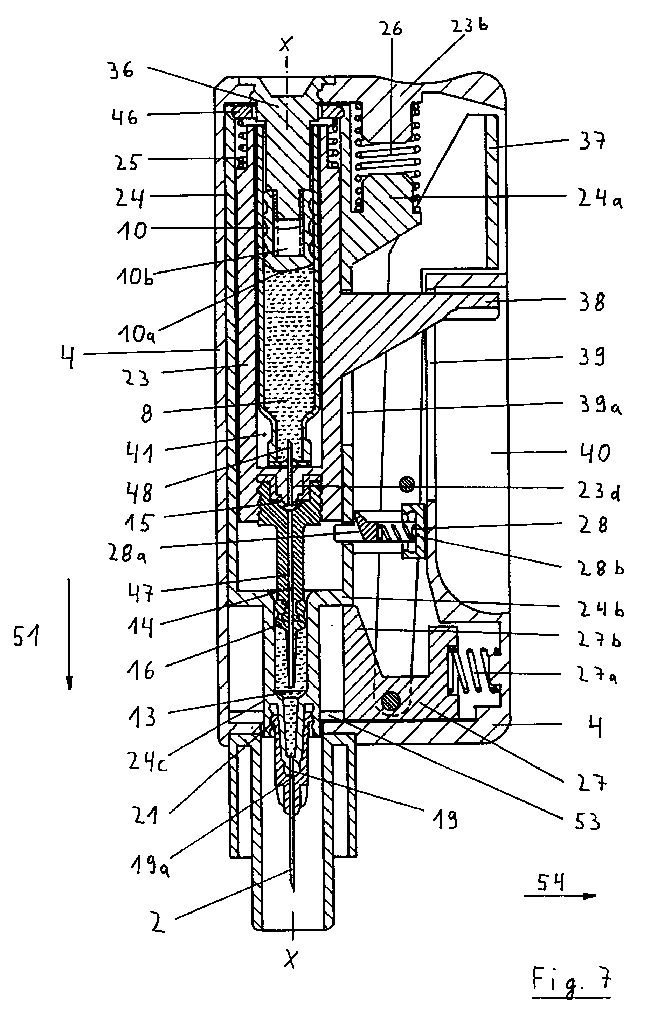

In Figur 3 sind schematisch das Einführen der Glascarpule 8 in die Vorrichtung 3 und die Anordnung des Verschlußelementes 18 sowie die Anordnung einer Kanüle 2 abgebildet. Der Deckel 36 wird zur Einführung der Glascarpule zunächst unter Freigabe des innerhalb des Gehäuses 4 angeordneten Bereiches 41 zur Aufnahme des mit dem flüssigen pharmazeutischen Produkt gefüllten Gefässes, entfernt. Nachdem die Glascarpule 8 in den Bereich 41 eingeführt ist, wird die entsprechende Öffnung wieder mit dem Deckel 36 verschlossen. Fußseitig weist der Deckel 36 einen sich axial erstreckenden Verlängerungsstift 42 auf, der in einem Endabschnitt 42 a endet. Der Verlängerungsstift 42 und der Endabschnitt 42 a sind mit dem Deckel 36 axial verschiebbar. Dieser Endabschnitt 42 a ist mit einer in einem Stopfen 10 entsprechend ausgebildeten Vertiefung in Eingriff bringbar, wodurch eine axial nach unten gerichtete Kraft auf die Carpule 8 ausübbar ist, sobald die Carpule 8 in dem Bereich 41 angeordnet und der Deckel 36 auf die entsprechende Öffnung aufgeschraubt oder mittels eines Bajonettverschlusses fixiert wird. Bei einer anderen, nicht gezeigten bevorzugte Ausführungsform sind der Verlängerungsstift 42 und der Endabschnitt 42 a relativ zum Deckel 36 axial in Pfeilrichtung 51 verschiebbar, so daß nach Anbringung des Deckels 36 am Gehäuse 4 eine von der Art der Befestigung des Deckels 36 unabhängige axiale Verschiebung des Verlängerungsstiftes 42 und des Endabschnittes 42 a manuell vom Benutzer herbeigeführt werden kann. Dies wird dadurch erreicht, daß der Benutzer bei aufgesetztem Deckel 36 eine Kraft auf den über den Deckel 36 nach außen vorstehenden Abschnitt des Verlängerungsstiftes 42 ausübt. Fußseitig ist die mit dem flüssigen pharmazeutischen Produkt gefüllte Glascarpule 8 mit einer Durchstechmembrane 9 verschlossen, die ihrerseits durch eine Umbördelung in Verbindung mit einem Metallband 44 in üblicher Weise gehaltert wird. An einem Anbringungsabschnitt 21 (beispielsweise in Figur 7 gezeigt) ist wahlweise eine Kanüle 2 oder das Verschlußelement 18 über ein entsprechendes Gewinde fixierbar, wie diese beiden Möglichkeiten in Figur 3 gezeigt werden. Hierbei ist die Kanüle 2 von einem Kanülenschutz 45 umgeben, durch die die Kanüle 2 vom Benutzer gehandhabt werden kann, ohne daß dieser sich dabei verletzt. Der Kanülenschutz 45 ist auf den Sockelbereich 2 a der Kanüle 2 aufklemmbar und ggf. über ein Gewinde am Sockelbereich 2 a fixierbar.

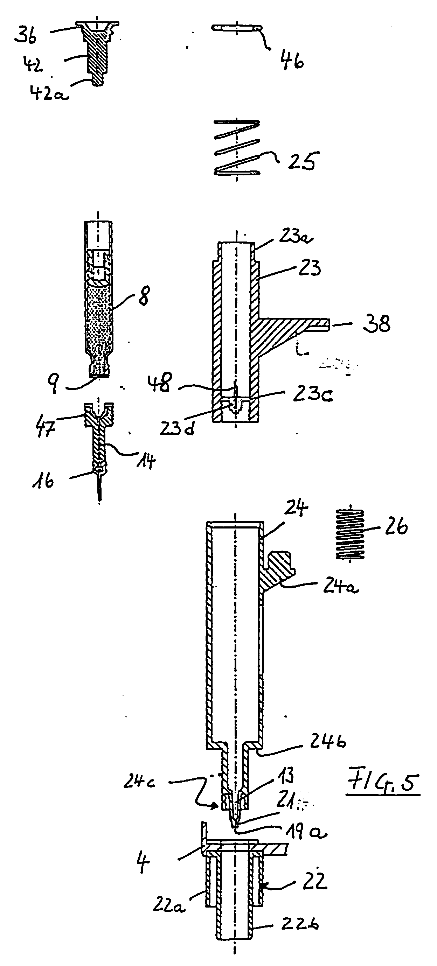

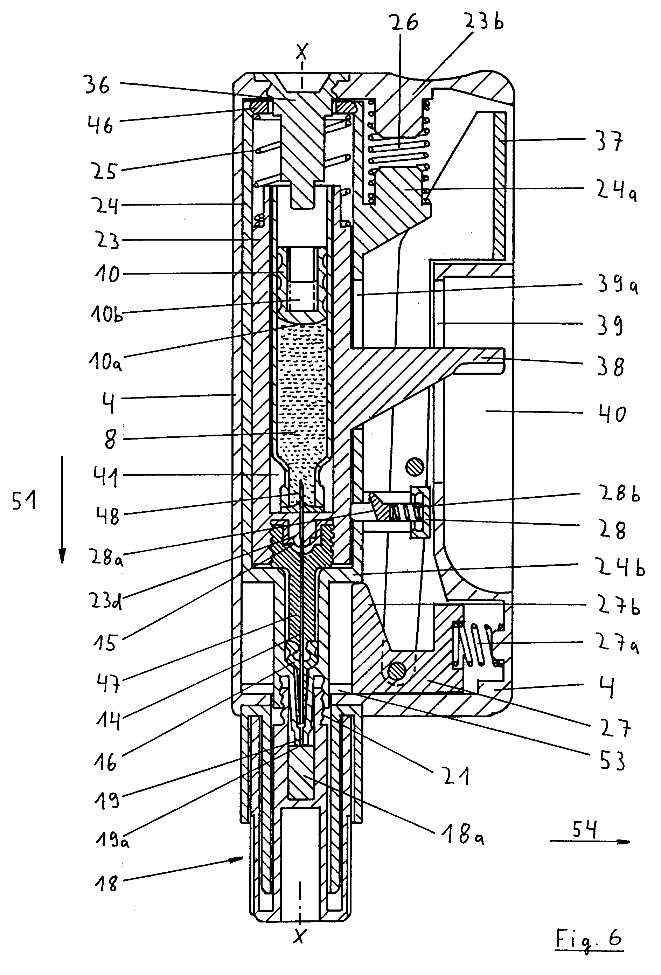

Das zylindrische Verschlußelement 18 (Figur 4) weist ein Dichtungselement 18 a auf, das mit einer Kanalmündung 19 (Figur 5 und 6) in Eingriff bringbar ist und durch das die Kanalmündung 19 flüssigkeits- und gasdicht verschließbar ist, sobald das Verschlußelement 18 über ein entsprechendes Gewinde 18 b an dem Anbringungsabschnitt 21 fixiert ist. Desweiteren weist das Verschlußelement 18 einen Schlitzbereich 18 c zur Aufnahme der Wandung des inneren Zylinders 22 b des Schutzelementes 22 auf.

In der schematischen Explosionsansicht der Figur 5 sind die wesentlichen inneren Teile der Vorrichtung 3 abgebildet. Hierzu gehören ein erster, innerer Zylinder 23, ein zweiter, äußerer Zylinder 24, wobei der zweite Zylinder 24 endseitig in dem Anbringungsabschnitt 21 endet, an den wahlweise ein Kanüle 2 oder das Verschlußelement 18 arretierbar ist. In diesem Bereich ist am Gehäuse 4, von dem in Figur 5 nur ein Teilbereich angedeutet ist, das Schutzelement 22 lösbar befestigt. Dem ersten Zylinder 23 ist eine erste Feder 25 und dem zweiten Zylinder 24 ist eine zweite Feder 26 zugeordnet, wobei sich die erste Feder 25 zwischen einem oberen Bereich 23 a des ersten Zylinders 23 und einem am zweiten Zylinder 24 festgelegten Ringelement 46 erstreckt, während sich die zweite Feder 26 von einem unteren Lager 24 a am zweiten Zylinder 24 und einem oberen Lager 23 b am Gehäuse 4 abstützt. Einstückig mit dem ersten Zylinder 23 ist das Verschiebeelement 38 ausgebildet.

Desweiteren weist der erste Zylinder 23 eine Bodenplatte 23 c auf, die fußseitig mit einem Vorsprung 23 d versehen ist. Mit diesen Vorsprung ist der Schaft 47 eines Kolbens 16 verbindbar, so daß der Kolben 16 an dem ersten Zylinder 23 fixiert ist und zusammen mit diesem bewegt wird. Innerhalb des Schaftes 47 sowie des Kolbens 16 verläuft ein Verbindungskanal 14, wobei dieser Verbindungskanal 14 auch durch die Bodenplatte 23 c sowie durch den Vorsprung 23 d geführt ist und in einer Hohlnadel 48 mündet.

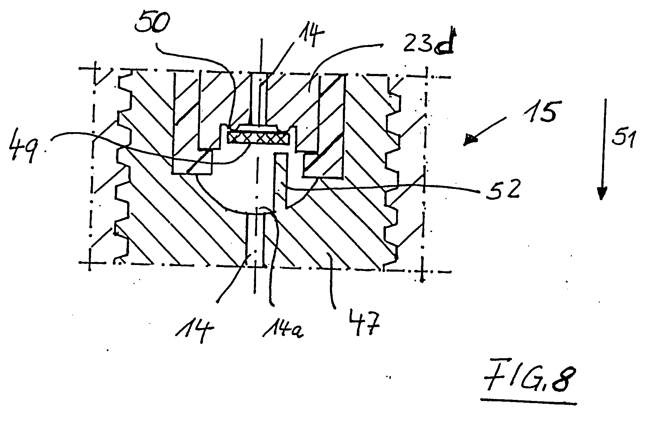

Wird nunmehr die mit dem flüssigen pharmazeutischen Produkt gefüllte Glascarpule 8 in den Innenraum, der den Bereich 41 zur Aufnahme des Gefässes ausbildet, des ersten Zylinders 23 eingesetzt und hierbei auf die Glascarpule mittels des Stopfens und des Verlängerungsstiftes 42 bzw. des Endabschnittes 42 a ein zum Anbringungsabschnitt 21 gerichteter Druck ausgeübt, so durchsticht die Hohlnadel 48 die Durchstechmembrane 9 der Glascarpule 8, so daß der Verbindungskanal 14 an den Inhalt der Glascarpule 8 angeschlossen wird. Hierbei ist dem Verbindungskanal 14 im Bereich der Verbindung des Schaftes 47 mit dem Vorsprung 23 d ein Rückschlagventil 15 zugeordnet, wobei dieses Rückschlagventil 15 in Figur 8 vergrößert abgebildet ist.

Dieses Rückschlagventil 15 weist gemäß Figur 8 eine scheibenförmige, runde Dichtungsmembrane 49 auf, die von einem Ringlager 50 getragen wird. Unterhalb der Dichtungsmembrane 49 ist ein von der Flüssigkeit durchströmter und die Kanaleinmündung 14 a des Kanals 14 erweiternder Bereich ausgebildet. Bei einer Strömungsrichtung der Flüssigkeit in Pfeilrichtung 51, wie diese bei der Auffüllung des Zwischenspeichers im Kanal 14 erforderlich ist, wird die Dichtungsmembrane 49 vom Ringlager 50 abgehoben und behindert den Durchfluß der Flüssigkeit nicht. Hierbei wird diese Bewegung der Dichtungsmembrane durch vier sternförmig angeordnete Stegelemente 52 begrenzt, so daß verhindert wird, daß die Dichtungsmembrane 49 die erweiterte Kanaleinmündung 14 a verschließt. Bei einer Strömungsrichtung der Flüssigkeit entgegengesetzt zur Pfeilrichtung 51 wird die Dichtungsmembrane 49 in eine Stellung gebracht, in der sie dichtend auf dem Ringlager 50 aufliegt, so daß hierdurch eine Strömungsrichtung entgegengesetzt zur Pfeilrichtung 51 verhindert wird, ohne daß dabei die Gefahr besteht, daß die Dichtungsmembrane 49 festklebt.

In den Figuren 6 und 7 sind die selben Ausführungsformen der Vorrichtung, jedoch in unterschiedlichen Betriebszuständen, dargestellt. Hierbei bildet die Figur 6 die Vorrichtung in einem Zustand ab, in der der Zwischenspeicher 13 entleert ist, wobei in diesem Zustand die Einzeldosis des flüssigen pharmazeutischen Produktes somit parenteral appliziert wurde und in der der jeweilige Benutzer die Vorrichtung vorfindet, um sie für die nächste Einzeldosis vorzubereiten. In der Figur 7 ist der Zwischenspeicher 13 mit einer Einzeldosis des aus der Carpule 8 über den Kanal 14 zugeführten flüssigen pharmazeutischen Produkt gefüllt. Desweiteren ist bei dem in Figur 7 gezeigten Betriebszustand am Anbringungsabschnitt 19 eine Kanüle 2 befestigt, so daß die Figur 7 insgesamt die Vorrichtung in einer Betriebsweise abbildet, wie diese unmittelbar vor der parenteralen Applikation der Einzeldosis des flüssigen pharmazeutischen Produktes durch den Benutzer vorliegt.

Die in Figur 5 gezeigten und vorstehend beschriebenen Teile sind in den Figuren 6 und 7 ebenfalls enthalten und dort mit den selben Bezugsziffern versehen.

In dem ersten Zylinder 23 ist in dem dafür vorgesehenen Bereich 41 die Carpule 8 angeordnet, während der zweite Zylinder 24 in seinem unteren Bereich 24 c den Zwischenspeicher 13 ausbildet (auch Figur 5).

Ausgehend von der in Figur 6 gezeigten Betriebsweise, in der das Verschlußelement 18 an dem Anbringungsabschnitt 21 arretiert ist und der das Dichtungselement 18 a die Kanalmündung 19 a des Kanales 19, der den Zwischenspeicher mit der Kanalmündung verbindet, verschließt, sind der erste Zylinder 23 und der zweite Zylinder 24 gemeinsam durch eine axiale Verschiebung der beiden Zylinder entgegengesetzt zur Pfeilrichtung 51 und relativ zum Gehäuse 4 aus einer nicht abgebildeten ersten Stellung, in der die Feder 25 weitestgehend entspannt ist und in der der zweite Zylinder 24 auf einem Gehäuseabschnitt 53 mittels des Rücksprunges 24 b (Figur 5) aufliegt, in eine zweite Stellung gebracht worden, wie diese in Figur 6 abgebildet ist. In dieser zweiten Stellung ist somit die in der ersten Stellung der beiden Zylinder entspannten Feder 25 gespannt, wobei eine Fixierung dieser zweiten Stellung durch eine am Rücksprung 24 b angreifende zweite Raste 27 erfolgt.

Diese axiale gemeinsame Verschiebung der beiden Zylinder 23 und 24 erfolgt durch die manuelle Betätigung des außen am Gehäuse angeordneten Verschiebeelementes 38, wobei diese axiale Verschiebung des Verschiebeelementes 38 durch einen im Gehäuse 4 vorgesehenen Schlitz 39 (Figur 3) geführt wird.

Um den entleerten Zwischenspeicher 13 in dieser zweiten Stellung nunmehr aus der innerhalb des ersten Zylinders 23 angeordneten Glascarpule 8 mit einer Einzeldosis des flüssigen pharmazeutischen Produktes zu befüllen, wird der erste Zylinder 23 entgegengesetzt zur Pfeilrichtung 51 und relativ zum Gehäuse 4 sowie dem zweiten Zylinder 24, der in der zweiten Stellung verbleibt, mittels des Verschiebeelementes 38 gegen die Kraft der zweiten Feder 26 in die dritte Stellung verschoben, wobei diese dritte Stellung axial entgegengesetzt zur Pfeilrichtung 51 verschoben, wobei diese dritte Stellung in der Figur 7 abgebildet ist.

In dieser dritten Stellung des ersten Zylinders wird der erste Zylinder 23 durch eine erste Raste 28 fixiert (Figur 7).

Bedingt dadurch, daß das Verschlußelement 18 aufgrund des fluiddichten Eingriffes seines Dichtungselementes 18 a mit der Kanalmündung 19 a ein Belüften des Zwischenspeichers 13 über den Kanal 19 verhindert, bildet sich im Zwischenspeicher 13 bei der axialen Verschiebung des ersten Zylinders 23 aus der zweiten Stellung in die dritte Stellung im Zwischenspeicher 13 ein Vakuum aus. Dies hängt damit zusammen, daß gleichzeitig mit der Verschiebung des ersten Zylinders 23 der hieran über den Kolbenschaft 47 befestigte Kolben 16 axial entgegengesetzt zur Pfeilrichtung 51 verschoben wird. Dieses im Zwischenspeicher anstehende Vakuum bewirkt dann, daß aus der Carpule 8 exakt eine Einzeldosis des flüssigen pharmazeutischen Produktes über den Verbindungskanal 14 und das im Vorsprung 23 d des ersten Zylinders angeordnete und in Figur 8 gezeigte geöffnete Rückschlagventil 15 der Zwischenspeicher vollständig gefüllt wird.

Diese axiale Verschiebung des ersten Zylinders aus der zweiten Stellung in die dritte Stellung relativ zum Gehäuse und dem zweiten Zylinder wird dadurch ermöglicht, daß in der Wandung des zweiten Zylinders eine schlitzförmige Führung 39 a vorgesehen ist, durch die sich ein Bereich des Verschiebeelementes 38, das einstückig mit dem ersten Zylinder 23 ausgebildet ist, erstreckt.

Nunmehr wird das Verschlußelement 18 gegen eine Kanüle 2 ausgetauscht, wobei die Kanüle 2 vom Benutzer am Kanülenschutz 45 gefaßt wird und die Kanüle 2 auf den Anbringungsabschnitt 21 aufgeschraubt wird. Hiernach entfernt der Benutzer den Kanülenschutz 45 und die Vorrichtung ist nunmehr bereit, die Einzeldosis des flüssigen pharmazeutischen Produktes zu applizieren.

Zu diesem Zweck wählt der jeweilige Benutzer einen Körperbereich aus und bringt die untere Zone des inneren Zylinders 22 b des Schutzelementes 22 in Anlage an diesen Körperbereich. Durch Betätigung der Auslösetaste 37 wird die zweite Raste 27 entgegen der Federkraft 27 a in Pfeilrichtung 54 verschoben, so daß der erste Zylinder 23 gemeinsam mit dem zweiten Zylinder 24 in Pfeilrichtung 51 axial relativ zum Gehäuse 4 aufgrund der Feder 26 verschoben wird. Dies führt dazu, daß die Spitze der Kanüle 2 in die ausgewählte Körperzone des Benutzers eindringt. Ein an der zweiten Raste 27 vorgesehener Vorsprung 27 b tritt während der gemeinsamen axialen Verschiebung der beiden Zylinder 23 und 24 in Eingriff mit einem an der ersten Raste 28 angeordneten Vorsprung 28 a, was dazu führt, daß die erste Raste 28 entgegen der Federkraft der darin vorgesehenen Feder 28 b ebenfalls in Pfeilrichtung 54 verschoben wird. Hierdurch wird der erste Zylinder 23 aus seiner dritten Stellung freigegeben, so daß er ebenfalls in Pfeilrichtung 51 axial aufgrund der Feder 25 verschoben wird. Dies wiederum führt dazu, daß der über den Schaft 47 mit dem ersten Zylinder 23 verbundene Kolben 16 axial in Pfeilrichtung 51 verschoben wird und somit die in dem Zwischenspeicher 13 befindliche Einzeldosis des flüssigen pharmazeutischen Produktes über die Kanüle 2 in den Körperbereich injiziert. Die axiale Bewegung des ersten Zylinders 23 wird durch den Rücksprung 24 b des zweiten Zylinders 24 und die axiale Bewegung des zweiten Zylinders 24 durch den Gehäuseabschnitt 53 beendet.

Nach erfolgter parenteraler Applikation zieht der Benutzer die Kanüle mittels der Vorrichtung aus dem Körperbereich, setzt den Kanülenschutz 45 auf die Kanüle 2, schraubt die Kanüle vom Anbringungsabschnitt 21 ab und schraubt das Verschlußelement 18 auf den Anbringungsabschnitt 21 auf. Abhängig von der hierbei vom Benutzer ausgeübten Kraft verbleiben dabei entweder der erste und zweite Zylinder in der ersten Stellung oder beide Zylinder werden in eine Stellung überführt, wie diese in Figur 6 gezeigt ist.

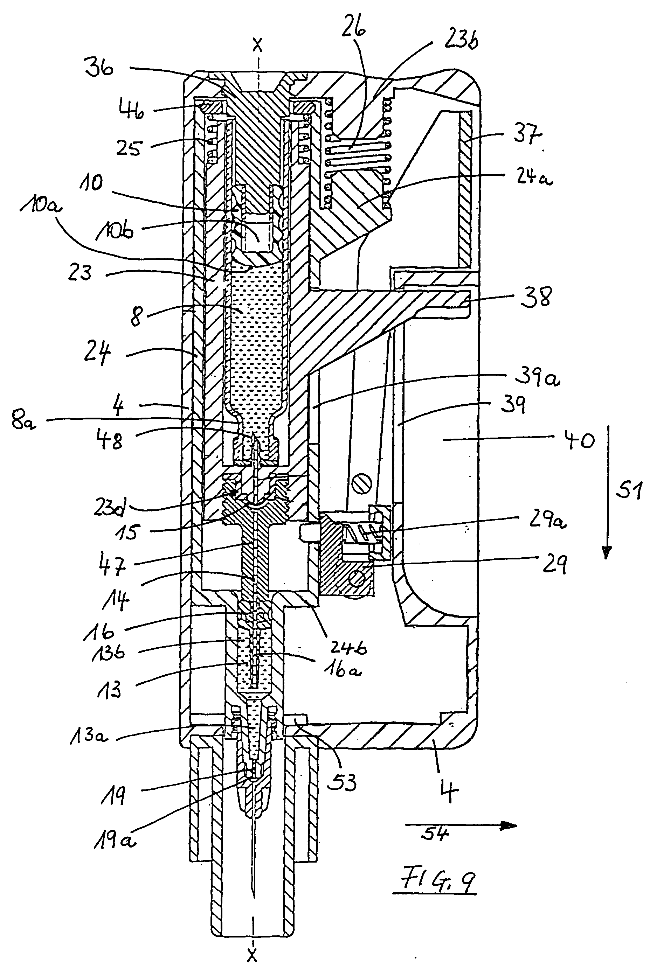

Die in Figur 9 gezeigte zweite Ausführungsform der Vorrichtung bildet die Vorrichtung in einer Betriebsweise ab, in der der Zwischenspeicher 13 mit der Einzeldosis des flüssigen pharmazeutischen Produktes gefüllt ist. Somit befindet sich die in Figur 9 gezeigte Ausführungsform in einem betriebsbereiten Zustand, d.h. unmittelbar vor der Injektion. Im Unterschied zu der zuvor anhand der Figuren 6 und 7 beschriebenen Ausführungsform sind bei der in Figur 9 gezeigten Ausführungsform die beiden Zylinder 23 und 24 nur zwischen einer ersten Stellung (nicht gezeigt), in der der erste Zylinder 23 am Rücksprung 24 b und der Rücksprung 24 b am Gehäuseabschnitt 53 anliegt und in der der Zwischenspeicher 13 entleert ist, und einer zweiten Stellung, wie diese in Figur 9 gezeigt ist und in der der Zwischenspeicher mit der Einzeldosis gefüllt ist, axial in Pfeilrichtung 51 und umgekehrt hierzu verschiebbar. Desweiteren ist bei der in Figur 9 gezeigten Ausführungsform nur eine einzige Raste 29 vorgesehen, die die Lage des ersten Zylinders und des zweiten Zylinders 24 in der zweiten Stellung fixiert und die über die Auslösetaste 37 lösbar ist.

Um bei der in Figur 9 gezeigten Ausführungsform der Vorrichtung die zeitliche Verzögerung zwischen dem Einstechvorgang der Kanüle in den Körperbereich des Benutzers und anschließend hiernach erst die Entleerung des Zwischenspeichers 13 und somit die eigentliche parenterale Applikation der Einzeldosis des flüssigen pharmazeutischen Produktes zu ermöglichen, sind die Federkennlinien der Federn 25 und 26 so aufeinander angepaßt, daß die Federkennlinie der Feder 26 steiler verläuft als die Federkennlinie der Feder 25. Bei einer Betätigung der Auslösetaste 37 verschiebt sich die Raste 29 entgegen der Federkraft der Feder 29 a in Pfeilrichtung 54, so daß die Raste 29 gelöst wird und somit gemeinsam der erste Zylinder 23 und der zweite Zylinder 24 in Pfeilrichtung 51 axial relativ zum Gehäuse verschoben werden. Aufgrund der steileren Federkennlinie der Feder 26 eilt der zweite Zylinder bei der axialen Verschiebung relativ zum ersten Zylinder, dessen Feder 25 die flachere Federkennlinie aufweist, dem ersten Zylinder 23 voraus, so daß die Kanüle 2 zuerst in die Körperzone eindringt und dann erst der Zylinder 23 und somit auch der mit dem Zylinder 23 über den Schaft 47 verbundene Kolben 16 die Einzeldosis des flüssigen pharmazeutischen Produktes aus dem Zwischenspeicher 13 heraus in den Körperbereich des Benutzers überführt.

Um bei den zuvor beschriebenen Ausführungsformen der erfindungsgemäßen Vorrichtung ein restloses Entleerens des Zwischenspeichers sicherzustellen, weist der Zwischenspeicher 13 einen zylindrischen Abschnitt 13 b und einen sich hieran anschließenden konischen Abschnitt 13 a auf (Figur 9), wobei die Ausflußseite des konischen Abschnitts 13 a in den Kanal 19 mündet, wie dies in Figur 9 am besten zu erkennen ist. Dementsprechend weist der im Zwischenspeicher 13 axial verschiebbare Kolben 16 einen zylindrischen Kolbenbereich und einen zum Kanal 19 weisenden konischen Kolbenbereich 16 a auf, wobei dieser konische Kolbenbereich durch ein entsprechend konisch geformtes Mantelrohr gebildet wird, daß die Auslaufseite des Verbindungskanals 14 umschließt. Fußseitig ist dieses Mantelrohr verschlossen und weist auf seiner konischen Mantelfläche Austrittsöffnungen auf, so daß die über den Kanal 14 zugeführte Flüssigkeit über diese Austrittsöffnungen in den Zwischenspeicher gelangt. Bei einer axialen Absenkung dieses Kolbens in Pfeilrichtung 51 verdrängt somit der konische Kolbenabschnitt 16 a die im Zwischenspeicher 13 befindliche Flüssigkeit dadurch, daß der konische Kolbenabschnitt 16 a in den konischen Zylinderabschnitt 13 a eindringt und diesen vollständig ausfüllt.

Ebenso ist es möglich, einen Zwischenspeicher 13 vorzusehen, der den zuvor beschriebenen und insbesondere in der Figur 9 gezeigten konischen Abschnitt 13 a überhaupt nicht oder nur in einer begrenzten axialen Länge aufweist. Mit anderen Worten mündet somit hierbei der zylindrische Abschnitt 13 b des Zwischenspeichers 13 direkt in den Kanal 19, oder der zylindrische Abschnitt 13 b ist über einen entsprechend verkürzten konischen Abschnitt 13 a mit dem Kanal verbunden. Dementsprechend ist der im Zwischenspeicher 13 vorgesehene Kolben 16 an die Form eines derartigen Zwischenspeichers angepaßt, wobei insbesondere eine dem Kolbenmantel zugeordnete Dichtung, vorzugsweise ein O-Ring aus einem gummielastischem Material, eine einwandfreie Betriebsweise der Vorrichtung 3 sicherstellt.

Bei allen zuvor gezeigten und beschriebenen Ausführungsformen wird mit zunehmender Entleerung der Glascarpule 8 der darin angeordnete Stopfen 10 ebenfalls in Pfeilrichtung 51 aufgrund des in der Glascarpule sinkenden Flüssigkeitsniveaus axial verschoben. Hierdurch wird verhindert, daß sich in der Glascarpule ein Vakuum oder oberhalb des Flüssigkeitsniveaus ein Gaspolster ausbildet. Um desweiteren eine restlose Entleerung der Glascarpule 8 sicherzustellen, verjüngt sich die Stirnfläche 10 a (Figur 9) des Stopfens 10, so daß sie bei vollständig abgesunkenem Flüssigkeitsstand an den Halsbereich der Glascarpule 8 a (Figur 9) anliegt.

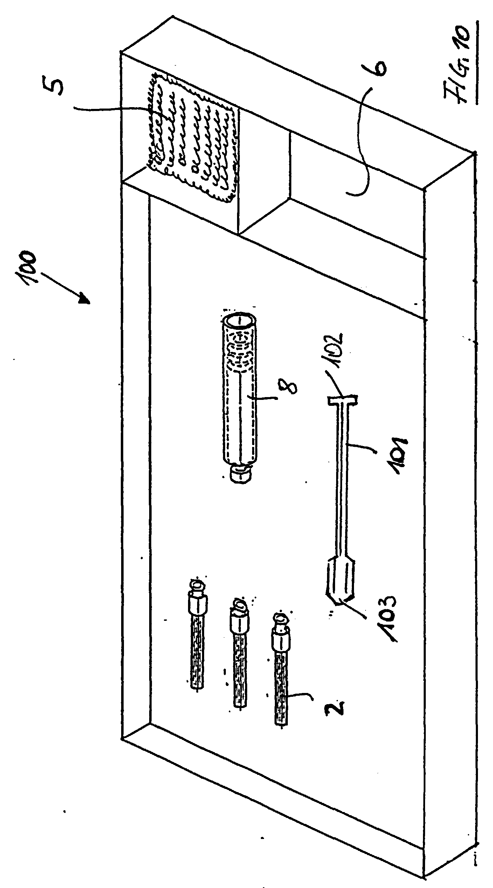

In diesem Zustand kann die vollständig entleerte Glascarpule mit dem in Figur 10 gezeigten Demontageelement 101 aus der Vorrichtung entfernt werden. Zu diesem Zweck weist das Demontageelement einen Griffabschnitt 102 zur Erfassung des Demontageelementes 101 sowie einen Arretierungsabschnitt, der bei entferntem Stopfen 36 in kraftschlüssigen Eingriff bringbar ist mit einer im Stopfen 10 vorgesehenen Aussparung 10 b (Figur 9).

Desweiteren umfaßt die in Figur 10 abgebildete Nachfülleinheit 100 eine Vielzahl von Kanülen 2, die mit einem entsprechendem Kanülenschutz 45 (Figur 3) zur sterilen Aufbewahrung versehen sind, eine mit 8 bezeichnete Glascarpule, die als mit einem flüssigen pharmazeutischen Produkt gefülltes Gefäß dient. Desweiteren sind innerhalb der Nachfülleinheit 100 eine der Anzahl der Kanülen 2 entsprechende Anzahl von mit einem Desinfektionsmittel genetzte Tupfer 5 vorhanden. Ferner weist die Nachfülleinheit 100 einen Bereich 6 zur Aufnahme von benutzten Kanülen auf, wobei dieser Bereich bei der in der Figur 10 abgebildeten Ausführungsform mit einem nicht gezeigten Deckel verschließbar ist. Insgesamt ist dieser Bereich 6 lösbar an der Nachfülleinheit 100 angeordnet, so daß nach Aufnahme aller benutzten Kanülen dieser Bereich 6 getrennt entsorgt werden kann, sofern dies gesetzlich vorgeschrieben oder erwünscht ist. In der Nachfülleinheit 100 sind üblicherweise 10 Kanülen 2 enthalten, so daß dementsprechend die Flüssigkeitsmenge für 10 Einzeldosen in der Glascarpule 8 enthalten ist. Üblicherweise variiert die in jeder Einzeldosis abgegebene Flüssigkeitsmenge zwischen 0,1 ml und 1 ml, so daß dementsprechend in der Glascarpule zwischen 1 ml und 10 ml des flüssigen pharmazeutischen Produktes enthalten ist. Um zu vermeiden, daß das Desinfektionsmittel der Tupfer 5 verdampft, ist jeder Tupfer 5 separat und fluiddicht in einer entsprechenden Kunststoffolie verpackt. Die Nachfülleinheit 100 ist insgesamt mit einem Deckel (nicht gezeigt) verschlossen oder ist unter Aufnahme der zuvor beschriebenen Teile in der üblichen Kunststoffverpackung, ggf. sogar steril, angeordnet. Ferner ist in der Nachfülleinheit 100 das zuvor beschriebene Demontageelement 101 vorgesehen.

Wiederholt wurde vorstehend der Begriff Durchstechmembrane verwendet, wobei hierunter insbesondere eine Dichtungsscheibe aus einem gummielastischem Material verstanden wird, die über eine Bördelkappe, vorzugsweise eine metallische Bördelkappe, an der Carpule arretierbar ist, wie dies in der Zeichnung deutlich erkennbar ist.

## Patentansprüche

1. Verkaufseinheit für die parenterale Applikation von flüssigen pharmazeutischen Produkten durch den Benutzer, dadurch gekennzeichnet, daß in der Einheit (1) mindestens eine Kanüle (2), eine Vorrichtung (3) zum Ausgeben einer vorgegebenen Flüssigkeitsmenge des pharmazeutischen Produktes in Einzeldosen sowie mindestens ein mit dem flüssigen pharmazeutischen Produkt gefülltes Gefäß (8), das in die Vorrichtung (3) einsetzbar und mittels der Vorrichtung (3) in Einzeldosen entleerbar ist, enthalten ist, wobei jede Einzeldosis der vorgegebenen Flüssigkeitsmenge des pharmazeutischen Produktes entspricht.

2. Verkaufseinheit nach Anspruch 1, dadurch gekennzeichnet, daß die Einheit (1) eine der Zahl der Einzeldosen entsprechende Anzahl von Kanülen (2) aufweist.

3. Verkaufseinheit nach Anspruch 1 oder 2, dadurch gekennzeichnet, daß die Einheit (1) eine der Anzahl der Kanülen (2) entsprechende Anzahl von mit einem Desinfektionsmittel genetzten Tupfer (5) enthält.

4. Verkaufseinheit nach einem der vorangehenden Ansprüche, dadurch gekennzeichnet, daß die Einheit (1) einen Bereich (6) zur Aufnahme von benutzten Kanülen (2) aufweist.

5. Verkaufseinheit nach einem der vorangehenden Ansprüche, dadurch gekennzeichnet, daß das mit dem flüssigen pharmazeutischen Produkt gefüllte Gefäß (8) eine zylindrische oder zylinderartige Glascarpule (8) ist.

6. Verkaufseinheit nach Anspruch 5, dadurch gekennzeichnet, daß die Glascarpule (8) fußseitig mit einer Durchstechmembrane (9) und kopfseitig mit einem Stopfen (10) verschlossen ist, wobei der Stopfen (10) derart ausgebildet ist, daß er axial innerhalb der Glascarpule (8) mit abnehmendem Flüssigkeitsniveau in Richtung auf die Durchstechmembrane (9) verschiebbar ist.

7. Verkaufseinheit nach Anspruch 5 oder 6, dadurch gekennzeichnet, daß der Stopfen (10) auf seiner Mantelfläche mindestens eine, vorzugsweise zwei bis fünf, radial nach außen weisende scheibenförmige Abschnitte aufweist, die beim Einsetzen des Stopfens (10) in die Glascarpule (8) unter Ausbildung einer an der Mantelinnenfläche der Carpule (8) flüssigkeitsdicht und luftdicht anliegenden Dichtfläche verformbar sind.

8. Verkaufseinheit nach einem der Ansprüche 6 bis 8, dadurch gekennzeichnet, daß der Stopfen (10) innerhalb der Glascarpule (8) mit einer Kraft kleiner als 10 N, vorzugsweise mit einer Kraft zwischen 4 N und 8 N, axial verschiebbar ist.

9. Verkaufseinheit nach einem der vorangehenden Ansprüche, dadurch gekennzeichnet, daß das Gefäß (8) mit einer wäßrigen Lösung eines Heparins, vorzugsweise mit einer wäßrigen Lösung eines niedermolekularen Heparins und insbesondere mit einer wäßrigen Lösung eines Enoxaparin-Natriums, gefüllt ist.

10. Verkaufseinheit nach einem der vorangehenden Ansprüche, dadurch gekennzeichnet, daß das Gefäß (8) mit einer Lösung eines Wachstumshormones, vorzugsweise mit einer wäßrigen Lösung des Wachstumshormones, gefüllt ist.

11. Vorrichtung zum Ausgeben einer vorgegebenen Flüssigkeitsmenge eines pharmazeutischen Produktes, dadurch gekennzeichnet, daß die Vorrichtung (3) zum Ausgeben einer vorgegebenen Flüssigkeitsmenge in Einzeldosen einen innerhalb eines Gehäuses (4) angeordneten Bereich (41) zur Aufnahme des mit dem flüssigen pharmazeutischen Produkt gefüllten Gefässes (8) sowie einen Abgabebereich für die Einzeldosis des pharmazeutischen Produktes aufweist und daß das Gefäß (8) mit dem Abgabebereich über einen Zwischenspeicher (13) verbindbar ist, derart, daß im eingesetzten Zustand des Gefässes (8) die vorgegebene Flüssigkeitsmenge einer jeden Einzeldosis zunächst aus dem Gefäß (8) in den Zwischenspeicher (13) und dann aus dem Zwischenspeicher (13) in den Abgabebereich überführbar ist, wobei über das Volumen des Zwischenspeichers (13) die Menge der abgegebenen Flüssigkeit für jede Einzeldosis bestimmbar ist.

12. Vorrichtung nach Anspruch 11, dadurch gekennzeichnet, daß zwischen dem eingesetzten Gefäß (8) und dem Zwischenspeicher (13) ein Verbindungskanal (14) vorgesehen ist.

13. Vorrichtung nach Anspruch 12, dadurch gekennzeichnet, daß dem Verbindungskanal (14) ein Ventil (15) zugeordnet ist, das einen Flüssigkeitsstrom vom Zwischenspeicher (13) zurück zum Gefäß (8) verhindert.

14. Vorrichtung nach einem der Ansprüche 11 bis 13, dadurch gekennzeichnet, daß dem Zwischenspeicher (13) ein innerhalb des Zwischenspeichers vorgesehener und axial in Richtung auf den Abgabebereich verschiebbarer Kolben (16) zur Förderung einer jeden Einzeldosis des flüssigen pharmazeutischen Produktes zum Abgabebereich hin zugeordnet ist.

15. Vorrichtung nach einem der Ansprüche 11 bis 14, dadurch gekennzeichnet, daß der Zwischenspeicher (13) durch eine axiale Verschiebung des Kolbens (16) in Richtung auf das Gefäß (8) mit einer Einzeldosis des pharmazeutischen Produktes füllbar ist.

16. Vorrichtung nach Anspruch 15, dadurch gekennzeichnet, daß durch die axiale Verschiebung des Kolbens (16) in dem Zwischenspeicher (13) ein Vakuum in dem Zwischenspeicher (13) zur Überführung einer Einzeldosis des flüssigen pharmazeutischen Produktes aus dem Gefäß (8) in den Zwischenspeicher (13) einstellbar ist.

17. Vorrichtung nach Anspruch 16, dadurch gekennzeichnet, daß der Zwischenspeicher (13) mit dem Abgabebereich über einen Kanal (19) verbunden ist und daß der Kanal (19) durch ein Verschlußelement (18) luftdicht verschließbar ist.

18. Vorrichtung nach Anspruch 16 oder 17, dadurch gekennzeichnet, daß das Verschlußelement (18) als ein dem Kanal (19) zugeordnetes lösbares Verschlußelement (18) ausgebildet ist, derart, daß ein Dichtungsbereich (18 a) des Verschlußelementes (18) die Kanalmündung (19 a) luftdicht abdeckt.

19. Vorrichtung nach einem der Ansprüche 14 bis 17, dadurch gekennzeichnet, daß durch eine axiale Verschiebung des Kolbens (16) innerhalb des Zwischenspeichers (13) in Richtung auf den Abgabebereich der Zwischenspeicher (13) vollständig entleerbar ist.

20. Vorrichtung nach einem der Ansprüche 11 bis 19, dadurch gekennzeichnet, daß der Zwischenspeicher (13) zylindrisch ausgebildet ist und einen sich hieran anschließenden und zum Abgabebereich weisenden konisch ausgestalteten Endbereich (13 a) aufweist, wobei der Endbereich (13 a) mit dem Abgabebereich über den Kanal (19) verbunden ist, und daß der Kolben (16) eine hieran angepaßte Form aufweist.

21. Vorrichtung nach einem der Ansprüche 11 bis 20, dadurch gekennzeichnet, daß der Abgabebereich einen Anbringungsabschnitt (21) zur lösbaren Befestigung einer Kanüle (2) aufweist, derart, daß der Kanal (19) mit der Kanüle (2) fluiddicht verbindbar ist.

22. Vorrichtung nach Anspruch 17, 18 oder 21, dadurch gekennzeichnet, daß am Anbringungsabschnitt (21) das Verschlußelement (18) befestigbar ist, derart, daß durch das Verschlußelement (18) der nach außen weisende Kanalabschnitt (19 a) fluiddicht verschließbar ist.

23. Vorrichtung nach einem der Ansprüche 11 bis 22, dadurch gekennzeichnet, daß dem Anbringungsabschnitt (21) ein Schutzelement (22) zugeordnet ist, daß zumindestens teilweise eine an dem Anbringungsabschnitt (21) arretierbare Kanüle (2) abdeckt.

24. Vorrichtung nach einem der Ansprüche 11 bis 23, dadurch gekennzeichnet, daß innerhalb des Gehäuses (4) ein erster Zylinder (23) und ein den ersten Zylinder (23) umschließender zweiter Zylinder (24) vorgesehen ist, daß der erste und der zweite Zylinder (23; 24) axial relativ zum Gehäuse (4) verschiebbar sind, daß innerhalb des ersten Zylinders (23) der Bereich (41) zur Aufnahme des Gefässes ausgebildet ist und innerhalb des zweiten Zylinders (24) der mit dem Gefäß (8) verbundene Zwischenspeicher (13) vorgesehen ist, derart, daß der erste und der zweite Zylinder (23; 24) zwischen einer ersten Stellung, in der die am Anbringungsabschnitt (21) arretierbare Kanüle (2) über das Schutzelement (22) hinaus vorsteht und in der der Zwischenspeicher (13) über den Kanal (19) und die hieran angeschlossene Kanüle (2) entleert ist, und einer zweiten Stellung, in der eine am Anbringungsabschnitt (21) arretierbare Kanüle (2) vom Schutzelement (22) abgedeckt ist und in der der Zwischenspeicher (13) mit der Einzeldosis gefüllt ist, und umgekehrt hierzu axial verschiebbar ist sind.

25. Vorrichtung nach Anspruch 24, dadurch gekennzeichnet, daß durch eine axiale Bewegung des ersten und des zweiten Zylinders (23; 24) relativ zum Gehäuse (4) aus der ersten Stellung in die zweite Stellung der Zwischenspeicher (13) mit einer Einzeldosis des flüssigen pharmazeutischen Produktes füllbar ist.

26. Vorrichtung nach Anspruch 25, dadurch gekennzeichnet, daß dem ersten und dem zweiten Zylinder (23; 24) jeweils eine Feder (25; 26) zugeordnet ist, derart, daß in der zweiten Stellung der Zylinder (23; 24) die Federn (25; 26) gespannt und in der ersten Stellung der Zylinder (23; 24) die Federn (25; 26) entspannt sind.

27. Vorrichtung nach einem der Ansprüche 24 bis 26, dadurch gekennzeichnet, daß beide Zylinder (23; 24) über ein einziges Rastelement (29) in der zweiten Stellung arretierbar sind.

28. Vorrichtung nach einem der Ansprüche 26 oder 27, dadurch gekennzeichnet, daß die dem zweiten Zylinder (24) zugeordnete Feder (26) eine steilere Federkennlinie aufweist als die Feder (25), die dem ersten Zylinder (23) zugeordnet ist.

29. Vorrichtung nach einem der Ansprüche 11 bis 23, dadurch gekennzeichnet, daß innerhalb des Gehäuses (4) ein erster Zylinder (23) und ein den ersten Zylinder (23) umschließender zweiter Zylinder (24) vorgesehen ist, daß der erste und der zweite Zylinder (23; 24) axial relativ zum Gehäuse (4) verschiebbar sind, daß innerhalb des ersten Zylinders (23) der Bereich (41) zur Aufnahme des Gefässes (8) ausgebildet ist und innerhalb des zweiten Zylinders (24) der mit dem Gefäß (8) verbundene Zwischenspeicher (13) vorgesehen ist, derart, daß der erste und zweite Zylinder (23; 24) gemeinsam aus einer ersten Stellung, in der die an dem Anbringungsabschnitt (21) arretierbare Kanüle (2) über das Schutzelement (22) vorsteht und in der der Zwischenspeicher (13) leer ist in eine zweite Stellung, in der die an dem Anbringungsabschnitt (21) arretierbare Kanüle (8) von dem Schutzelement (22) abgedeckt ist, und umgekehrt hierzu axial relativ zum Gehäuse (4) verschiebbar ist, daß zum Füllen des Zwischenspeichers (13) der erste Zylinder (23) relativ zur zweiten Stellung des zweiten Zylinders (24) in eine dritte Stellung axial weiter in Richtung auf das Gefäß (8) verschiebbar ist.

30. Vorrichtung nach Anspruch 29, dadurch gekennzeichnet, daß jedem Zylinder (23; 24) eine Feder (25; 26) zugeordnet ist, wobei eine erste Feder (25) die Lage des ersten Zylinders (23) in der dritten Stellung und eine zweite Feder (26) die Lage des zweiten Zylinders (24) in der zweiten Stellung vorspannt, und daß der erste Zylinder (23) in der dritten Stellung durch eine erste Raste (28) und der zweite Zylinder (24) in der zweiten Stellung durch eine zweite Raste (27) fixierbar ist.

31. Vorrichtung nach Anspruch 29 oder 30, dadurch gekennzeichnet, daß durch Betätigung einer gemeinsamen Auslösetaste (37) zunächst die zweite Raste (27) und hierzu zeitversetzt die erste Raste (28) lösbar ist.

32. Vorrichtung nach einem der Ansprüche 11 bis 31 zur Verwendung in einer Verkaufseinheit (1) nach einem der Ansprüche 1 bis 10.

33. Nachfülleinheit zur Verwendung bei der Verkaufseinheit oder der Vorrichtung nach einem der vorangehenden Ansprüche, dadurch gekennzeichnet, daß die Nachfülleinheit (100) mindestens eine Kanüle (2) sowie mindestens ein mit dem flüssigen pharmazeutischen Produkt gefülltes Gefäß (8), das in die Vorrichtung (3) einsetzbar und mittels der Vorrichtung (3) in Einzeldosen entleerbar ist, umfaßt.

34. Nachfülleinheit nach Anspruch 33, dadurch gekennzeichnet, daß in der Nachfülleinheit (100) ein Demontageelement (101) zur Entfernung eines entleerten Gefässes (8) aus der Vorrichtung (3) zum Ausgeben einer vorgegebenen Flüssigkeitsmenge in Einzeldosen vorgesehen ist.

35. Nachfülleinheit nach Anspruch 33 oder 34, dadurch gekennzeichnet, daß die Nachfülleinheit (100) eine der Zahl der Einzeldosen entsprechende Anzahl von Kanülen (2) und/oder mit Desinfektionsmittel genetzten Tupfer (5) enthält.

36. Nachfülleinheit nach einem der Ansprüche 33 bis 35, dadurch gekennzeichnet, daß die Nachfülleinheit (100) einen Bereich (6) zur Aufnahme von benutzten Kanülen (2) aufweist.

37. Nachfülleinheit nach einem der Ansprüche 33 bis 36, dadurch gekennzeichnet, daß das mit dem flüssigen pharmazeutischen Produkt gefüllte Gefäß (8) eine zylindrische oder zylinderartige Glascarpule (8) ist.

38. Nachfülleinheit nach Anspruch 37, dadurch gekennzeichnet, daß die Glascarpule (8) fußseitig mit einer Durchstechmembrane (9) und kopfseitig mit einem Stopfen (10) verschlossen ist, wobei der Stopfen (10) derart ausgebildet ist, daß er axial in der Glascarpule (8) mit abnehmendem Flüssigkeitsniveau in Richtung auf die Durchstechmembrane (9) verschiebbar ist.

39. Nachfülleinheit nach Anspruch 37 oder 38, dadurch gekennzeichnet, daß der Stopfen (10) auf seiner Mantelfläche mindestens eine, vorzugsweise zwei bis fünf, radial nach außen weisende scheibenförmige Lippendichtabschnitte aufweist, die beim Einsetzen des Stopfens (10) in die Glascarpule (8) unter Ausbildung einer an der Mantelinnenfläche der Carpule flüssigkeitsdicht anliegenden Dichtfläche verformbar sind.

40. Nachfülleinheit nach einem der Ansprüche 37 bis 39, dadurch gekennzeichnet, daß der Stopfen (10) innerhalb der Glascarpule (8) mit einer Kraft kleiner als 10 N, vorzugsweise mit einer Kraft zwischen 4 N und 8, axial verschiebbar ist.

41. Nachfülleinheit nach einem der Ansprüche 33 bis 40, dadurch gekennzeichnet, daß das Gefäß (8) mit einer wäßrigen Lösung eines Heparins, vorzugsweise mit einer wäßrigen Lösung eines niedermolekularen Heparins und insbesondere mit einer wäßrigen Lösung eines Enoxaparin-Natriums, gefüllt ist.

42. Nachfülleinheit nach einem der Ansprüche 33 bis 40, dadurch gekennzeichnet, daß das Gefäß (8) mit einer Lösung eines Wachstumshormones, vorzugsweise mit einer wäßrigen Lösung des Wachstumshormones, gefüllt ist.
